# EUROPEAN PATENT SPECIFICATION

(11) **EP 0 416 912 B1**
(45) Date of publication and mention of the grant of the patent: **06.12.1995**
(21) Application number: 90309745.9
(22) Date of filing: 05.09.1990
(51) Int. Cl.: A61M 5/36

(54) **Automatic tubing lock for ultrasonic sensor interface**
Automatischer Schlauchverschluss zum Verbinden mit einem Ultraschallsensor
Système de blocage d'un tube pour l'interfacer avec un capteur à ultrasons

(30) Priority: 05.09.1989 US 404027
(43) Date of publication of application: 13.03.1991
(73) Proprietor: IVAC MEDICAL SYSTEMS, Inc., San Diego, CA 92121-2733 (US)
(72) Inventor: Gorton, Lanny A., Sunland, California 91040 (US)
(74) Representative: Rees, David Christopher

(56) References cited:
- EP-A- 0 090 440
- EP-A- 0 293 592
- US-A- 4 114 144

## Description

The present invention relates generally to an ultrasonic system for detecting the presence of air in a fluid line, and more particularly to an apparatus which automatically loads the outlet tubing at the bottom of a disposable cassette into a notch in an ultrasonic sensor housing located on a main pump unit when the cassette is installed on the main pump unit.

In the past there have been two primary techniques which have been used to deliver drugs which may not be orally ingested to a patient. The first such technique is through an injection, or shot, using a syringe and needle which delivers a large dosage at relatively infrequent intervals to the patient. This technique is not always satisfactory, particularly when the drug being administered is potentially lethal, has negative side effects when delivered in a large dosage, or must be delivered more or less continuously to achieve the desired therapeutic effect. This problem results in smaller injections being given at more frequent intervals, a compromise approach not yielding satisfactory results.

The second technique involves administering a continuous flow of medication to the patient, typically through an IV bottle. Medication may also be delivered through an IV system with an injection being made into a complex maze of IV tubes, hoses, and other paraphernalia. With drop counters being used to meter the amount of bulk fluid delivered, many medications still end up being administered in a large dosage through an injection into the IV lines, although the medications may be diluted somewhat by the bulk fluid.

As an alternative to these two techniques of administering medication to a patient, the relatively recent addition of medication infusion pumps has come as a welcome improvement. Medication infusion pumps are used to administer drugs to a patient in small, metered doses at frequent intervals or, alternatively, in the case of some devices, at a low but essentially continuous rate. Infusion pump therapy may be electronically controlled to deliver precise, metered doses at exactly determined intervals, thereby providing a beneficial gradual infusion of medication to the patient. In this manner, the infusion pump is able to mimic a natural process whereby chemical balances are maintained more precisely by operating on a continuous time basis.

One of the requirements of a medication infusion system is dictated by the important design consideration of disposability. Since the portion of the device through which the medication is pumped must be sterile, in most applications of modern medication infusion equipment, some portions of the equipment are used only once and then disposed of, typically at regular intervals such as once daily. It is therefore desirable that the fluid pump portion of the infusion pump device should be disposable, with the fluid pump being designed as an attachable cassette which is of inexpensive design, and which is easily installable onto the main pump unit.

It is clearly desirable to have a simple disposable cassette design to minimise the cost of its construction, using the minimum number of parts necessary in its design. The design of the cassette should be mass produceable, and yet result in a uniform cassette which is capable of delivering liquid medication or other therapeutic fluids with a high degree of accuracy. The cassette should include more than just a fluid pump; other features which have formerly been included in peripheral devices should also be included in the cassette.

Such a system has been disclosed in the present Applicants' co-pending applications published under numbers EP-A-321120, EP-A-319269, EP-A-319279, EP-A-319278, Ep-A-319276, EP-A-320168, EP-A-319273, EP-A-319274, EP-A-319268, EP-A-319277, EP-A-319275, EP-A-341346, EP-A-346548, EP-A-319272 and EP-A-319267.

An essential function of a medication infusion system is to avoid the infusion of fluid containing more than a minimal amount of air bubbles. Although steps may be taken to minimise the possibility of air bubbles being contained in a fluid which is to be infused to a patient, it is essential to monitor the fluid line before it reaches the patient to ensure that air bubbles remaining in the fluid to be infused are detected. The detection of air bubbles in all fluids which are to be infused is therefore a critical design requirement.

One type of air-in-line detector which has been used in the past is an ultrasonic detector, which employs an ultrasonic transmitter located on one side of a fluid line and an ultrasonic receiver located on the other side of the fluid line. Fluid is a good conductor of ultrasonic energy while air or foam is not. Accordingly, if there is an air bubble in the fluid line between the transmitter and the receiver, the signal strength will be greatly attenuated, and the presence of the bubble will be indicated. Examples of ultrasonic air-in-line detectors include US Patent No. 4,764,166, to Spani, and US Patent No. 4,821,558, to Pastrone et al.

The Pastrone et al device has a projection from a disposable cassette which fits into an ultrasonic detector. Thus, the fluid path monitored in the Pastrone et al device is within the fluid cassette. It is particularly desirable to monitor the fluid path as it leaves the cassette to determine whether or not there are air bubbles. The Spani device is designed to monitor fluid flow through a segment of flexible tubing, which may be the fluid path as it leaves the cassette.

The Spani device will work quite well to detect the presence of air bubbles in the tubing, but has at least two disadvantages in its operation. First, the tubing must be installed in the Spani device in a step additional to the installation of a cassette onto a main pump unit. In other words, there is no provision in the Spani device for the tubing to be installed automatically. This represents a disadvantage since an extra step is required by the operator of the system.

Secondly, the Spani device is extremely sensitive to the placement of the tubing in the sensor. If the tubing is moved slightly, the path of the ultrasonic energy could be disrupted, resulting in a false indication that there is air in the fluid line. Other than friction, there is no provision in the Spani device to retain the tubing in place in the sensor. Thus, while the Spani sensor operates well, it has certain disadvantages with regard to the installation and retention of the tubing in the sensor.

EP-A-90440 discloses a system for automatically loading a segment of tubing extending from a disposable cassette in to engagement with a sensor as the cassette is installed on to a pump unit. The pump unit comprises a main pump unit chassis defining a location into which the cassette may be installed and a sensor housing mounted integrally on the chassis downstream of the location where the cassette is to be installed.

It is therefore the primary object of the present invention to provide an ultrasonic air-in-line detection sensor for use with the outlet tubing from a cassette, with the tubing being automatically loaded into the proper position in the sensor when the cassette is installed on a main pump unit. The system of the present invention should preferably therefore enable the tubing to be loaded into the sensor, in the proper position, with no more effort required than the effort to load the cassette itself onto the main pump unit. It must be emphasised that the tubing has to be loaded correctly into the sensor in a highly repeatable manner.

It is also an object of the present invention that once the tubing has been properly loaded into the sensor, it should be retained in the sensor in such a way that the tubing will be highly unlikely to move with respect to the sensor. The tubing must thus be both properly loaded initially into the sensor, and retained in its proper position in the sensor to prevent false signals due to movement of the tubing in the sensor. It is a further object of the invention that the tubing be automatically removed from the sensor when the cassette is removed from the main pump unit without requiring any additional steps to be performed.

Despite the inclusion of all of the aforesaid features, it is a still further object of the present invention to employ a minimum number of parts, all of which are of inexpensive construction, yet which afford the assembled sensor and its mounting hardware the high degree of accuracy which must be retained. The system of the present invention should also be of a design which enables it to compete economically with known systems, and it should provide an ease of use rivalling the best of competing systems. The system should accomplish all these objects in a manner which will retain and enhance all of the advantages of reliability, durability, and safety of operation. The system of the present invention should provide all of these advantages and overcome the limitations of the prior art without incurring any relative disadvantage.

According to the present invention, there is provided a system for automatically loading a segment of tubing extending from a disposable, fluid-pumping cassette into engagement with a sensor as the cassette is installed onto a main pump unit, comprising a main pump unit chassis defining thereunder a location into which the cassette may be installed and a sensor housing mounted on the chassis downstream of the location into which the cassette may be installed, characterised in that the sensor housing has a vertically orientated slot located at the front for receiving the segment of tubing and a recess located in the bottom of the sensor housing on both sides of the slot in the sensor housing; the system further including a hollow cylindrical tubing adaptor adapted to be mounted on the segment of tubing extending from the bottom of the cassette, the top end of the tubing adaptor being tapered in order to be able to engage the recess in the bottom of the sensor housing when the cassette is inserted into the position defined by the main pump unit chassis.

According to a preferred variant, such a system comprises a main pump unit chassis defining a location where the cassette may be installed; a sensor-housing mounted on the main pump unit chassis adjacent the location where the cassette is to be installed, the sensor housing having a slot for receiving the segment of tubing; an ultrasonic transmitter located in the sensor housing on one side of the slot; an ultrasonic receiver located in the sensor housing on the other side of the slot; a recess located in the side of the sensor housing facing away from the location where the cassette is to be installed, the recess being located on both sides of the slot; and a hollow cylindrical tubing adaptor installed on the segment of tubing extending from the cassette, the end of the tubing adaptor nearest the cassette being tapered, the tubing adaptor being spaced away from the cassette so that as the cassette is inserted into its installation location defined by the main pump unit chassis, the tapered end of the tubing adaptor will engage the side of the sensor housing facing away from the cassette installation location, the tapered end of the tubing adaptor engaging the recess in the sensor housing to draw the segment of tubing extending from the cassette into the slot in the sensor housing when the cassette is inserted into position on the main pump unit chassis.

Preferably, there is an inclined ramp located on the side of the sensor housing facing away from the cassette installation location and in the front of the ultrasonic sensor housing on each side of the slot, with the ramps being inclined towards the recess in the ultrasonic sensor housing to urge the tapered end of the tubing adaptor into engagement with the recess in the sensor housing.

In a preferred embodiment, the cassette installation location is beneath the main pump unit chassis; the sensor housing is mounted on the main pump unit chassis below that location; the slot is at the front of the sensor housing and is vertically oriented; the recess is located in the bottom of the sensor housing on both sides of the slot; and the hollow cylindrical tubing adaptor extends from the bottom of the cassette, the top end of the tubing adaptor being tapered, the tubing adaptor being spaced away from the bottom of the cassette sufficiently far so that as the cassette is inserted into its installation location the tapered top end of the tubing adaptor engages the bottom of the sensor housing, the tapered top end of the tubing adaptor engaging the recess in the bottom of the sensor housing.

Preferably, the ultrasonic transmitter is adhesively mounted to the interior of the sensor housing on one side of the slot, and the ultrasonic receiver is adhesively mounted to the interior of the sensor housing on the other side of the said slot. Conveniently, the sensor housing comprises an upper sensor housing which is rectangular in configuration and has a vertically oriented opening at the front forming the upper portion of the slot, the upper housing being open at the bottom, and defining first and second areas in its interior on each side of the opening, a third area being defined by that portion of the interior of the upper housing into which the opening does not protrude; and a lower sensor housing located beneath the upper sensor housing, the lower sensor housing having an opening at the front forming the lower portion of the slot, the lower sensor housing sealing the open areas of the upper sensor housing. Thus, in such a case, the ultrasonic transmitter is located in the first area against the interior wall of first area facing the opening in the upper sensor housing, and the ultrasonic receiver is located in the second area against the interior wall of the second area facing the opening in the upper sensor housing.

There may be a first foam segment located between the ultrasonic transmitter and the interior wall of the first area remote from the opening in the upper sensor housing; and a second foam segment located between the ultrasonic receiver and the interior wall of the second area remote from the opening in the upper sensor housing.

The disadvantages and limitations of the background art discussed above are thus overcome by the present invention. With this invention, an ultrasonic transmitter and an ultrasonic receiver may be mounted in an ultrasonic sensor housing located below the position in which a disposable cassette will be mounted on a main pump unit. A notch or slot is formed in the front of the ultrasonic sensor housing to receive the outlet tubing, which extends from the bottom of the cassette. The ultrasonic transmitter is located in the ultrasonic sensor housing on one side of the notch, and the ultrasonic receiver is located in the ultrasonic sensor housing on the other side of the notch.

A recessed area is located on the bottom of the ultrasonic sensor housing around the notch. Two inclined ramps are located on the bottom and in the front of the ultrasonic sensor housing on each side of the notch, with the ramps being inclined towards the recessed area in the bottom of the ultrasonic sensor housing. A tubing adaptor is mounted on the outlet tubing below the cassette. The tubing adaptor is preferably a hollow cylinder with a tapered top end and a flange located below the tapered end.

When the cassette is positioned in front of the main pump unit prior to installation, the outlet tubing between the bottom of the cassette and the tubing adaptor is positioned in front of the notch in the ultrasonic sensor housing. As the cassette is installed onto the main pump unit, the outlet tubing between the bottom of the cassette and the tubing adaptor will move into the notch in the ultrasonic sensor housing. Simultaneously, the tapered end of the tubing adaptor will fit on the bottom of the ramps on the ultrasonic sensor housing.

As the cassette is moved into its final position on the main pump unit, the tapered end of the tubing adaptor will slide on the ramps on the ultrasonic sensor housing into the recessed area on the bottom of the ultrasonic sensor housing. This will pull the outlet tubing between the bottom of the cassette and the tubing adaptor fully into the notch in the ultrasonic sensor housing, orienting it directly between the ultrasonic transmitter and the ultrasonic receiver in the ultrasonic sensor housing.

Since the cassette is secured to the main pump unit, and the tubing adaptor is located in the recessed area on the bottom of the ultrasonic sensor housing, the outlet tubing between the bottom of the cassette and the tubing adaptor will be retained in the proper position as long as the cassette is locked into the main pump unit. This ensures both proper placement and retention of the portion of the outlet tubing between the bottom of the cassette and the tubing adaptor with respect to the ultrasonic transmitter and receiver. Thus, both installation and removal of the tubing into the ultrasonic sensor require only manipulation of the cassette.

It may therefore be appreciated that the present invention provides an ultrasonic air-in-line detection sensor for use with the outlet tubing from a cassette in which the tubing is automatically loaded into the proper position in the sensor when the cassette is installed on a main pump unit. The system of the present invention thus loads the tubing into the sensor, in the proper position, with no more effort required than the effort to load the cassette itself onto the main pump unit. The tubing is thereby loaded correctly into the sensor in a highly repeatable manner whenever the cassette is installed.

Once the tubing has been properly loaded into the sensor, it is retained in the sensor in a manner making it impossible for the tubing to move with respect to the sensor. The tubing is thus both properly loaded initially into the sensor, and retained in its proper position in the sensor to prevent false signals due to movement of the tubing in the sensor. In addition, the tubing is automatically removed from the sensor when the cassette is removed from the main pump unit without requiring any additional steps to be performed.

Despite the inclusion of all these features, the system of the present invention employs a minimum number of parts, all of which are of inexpensive construction, yet which afford the assembled cassette a high degree of accuracy. The system of the present invention is therefore of a design which enables it to compete economically with known systems, and it provides an ease of use rivalling the best of competing systems. The system accomplishes all these objects in a manner which retains and enhances the advantages of reliability, durability, and safety of operation. The system of the present invention provides these advantages and overcomes the limitations of the background art without incurring any relative disadvantage whatsoever.

The invention may be carried into practice in various ways and some embodiments will now be described by way of example with reference to the accompanying drawings. In this description of the preferred embodiments, a uniform directional system is sued in which front, back, top, bottom, left and right are indicated with respect to the operating position of the cassette and main pump unit when viewed from the front of the main pump unit. In the accompanying drawings:
Figure 1 is a top plan view of a disposable cassette body showing most of the fluid path through the cassette;
Figure 2 is a top plan view of a disposable cassette body shown in Figure 1;
Figure 3 is a rear-elevation (inverted) of the cassette body shown in Figures 1 and 2;
Figure 4 is a bottom view of the cassette body shown in Figures 1 to 3;
Figure 5 is a right side view (inverted) of the cassette body shown in Figures 1 to 4;
Figure 6 is a left side view of the cassette body showing in Figures 1 to 5;
Figure 7 is a partially cutaway view from the front of the cassette body shown in Figures 1 to 6, showing the bubble trap used to remove air bubbles from the fluid supplied to the cassette;
Figure 8 is a partially cutaway view from the right side (inverted) of the cassette body shown in Figures 1 to 6, showing the cylinder of the fluid pump contained in the cassette;
Figure 9 is a top plan view of a valve diaphragm used to seal the passageways on the top surface of the cassette body showing in Figure 1;
Figure 10 is a bottom view of the valve diaphragm shown in Figure 9;
Figure 11 is a vertical section through the valve diaphragm shown in Figures 9 and 10, viewed from the rear;
Figure 12 is a partially cutaway view from the right side of the valve diaphragm shown in Figures 9 and 10;
Figure 13 is a top plan view of a valve diaphragm retainer used to retain the valve diaphragm shown in Figures 9 to 12;
Figure 14 is a bottom view of the valve diaphragm retainer shown in Figure 13;
Figure 15 is a rear elevation of the valve diaphragm retainer shown in Figures 13 and 14;
Figure 16 is a front elevation of the valve diaphragm retainer shown in Figures 13 to 15;
Figure 17 is a right side view of the valve diaphragm retainer shown in Figures 13 to 16;
Figure 18 is a left side view of the valve diaphragm retainer shown in Figures 13 to 17;
Figure 19 is a vertical section through the valve diaphragm retainer shown in Figures 13 to 18, viewed from the front;
Figure 20 is a partially cutaway view from the left side of the valve diaphragm retainer shown in Figures 13 to 19;
Figure 21 is a partially cutaway view from the right side of the valve diaphragm retainer shown in Figures 13 to 20;
Figure 22 is a top view of a bubble chamber cap;
Figure 23 is a bottom view of the bubble chamber cap shown in Figure 22;
Figure 24 is a left side view of the bubble chamber cap shown in Figures 22 and 23;
Figure 25 is a cutaway view from the rear of the bubble chamber cap shown in Figures 22 to 24;
Figure 26 is a cutaway view (inverted) from the right side of the bubble chamber cap shown in Figures 22 to 24;
Figure 27 is a top plan view of a slide latch used both the lock the cassette in place on a main pump unit, and to pinch off the IV outlet line prior to installation on the main pump unit;
Figure 28 is a right side view of the slide latch shown in Figure 27;
Figure 29 is a bottom view of the slide latch shown in figures 27 and 28;
Figure 30 is a rear view of the slide latch shown in Figures 27 to 29;
Figure 31 is a front elevation of the slide latch shown in Figures 27 to 30;
Figure 32 is a cutaway view (inverted) from the left side of the slide latch shown in Figures 27 to 31;
Figure 33 is a side view of the piston cap and boot seal, which function both as a piston and as a bacterial seal;
Figure 34 is a view from above of the piston cap and boot seal shown in Figure 33;
Figure 35 is a view from below of the piston cap and boot seal shown in Figures 33 and 34;
Figure 36 is a vertical section through the piston cap and boot seal shown in Figures 33 to 35;
Figure 37 is a rear view of a piston for insertion into the piston cap and boot seal shown in Figures 33 to 36;
Figure 38 is a front view of the piston shown in Figure 37;
Figure 39 is a top view of the piston shown in Figures 37 and 38;
Figure 40 is a left side view of the piston shown in Figures 37 to 39;
Figure 41 is a bottom view of the piston shown in Figures 37 to 40;
Figure 42 is a cutaway view (inverted) from the right side of the piston shown in Figures 37 to 41;
Figure 43 is a perspective view from above of a tubing adaptor for installation in the outlet tube below the slide latch;
Figure 44 is a cutaway view of the tubing adaptor shown in Figure 43;
Figure 45 is a perspective view from above of an assembled cassette using the components shown in Figures 1 to 44, with the slide latch in the opened position;
Figure 46 is a bottom view of the assembled cassette shown in Figure 45, with the tubing adaptor removed for clarity and the slide latch in the opened position;
Figure 47 is a perspective view from above of the assembled cassette shown in Figures 45 and 46, with the slide latch in the closed position;
Figure 48 is a bottom view of the assembled cassette shown in Figures 45 to 47, with the tubing adaptor removed for clarity and the slide latch in the closed position;
Figure 49 is a left side view of the latch head used to capture and actuate the piston;
Figure 50 is a right side view of the latch head shown in Figure 49;
Figure 51 is a bottom view of the latch head shown in Figures 49 and 50;
Figure 52 is top view of the latch head shown in Figures 49 to 51;
Figure 53 is a cutaway view from the right side of the latch head shown in Figures 49 to 52;
Figure 54 is a right side view of the spring retainer to be mounted in the latch head shown in Figures 49 to 52;
Figure 55 is a front view of the spring retainer shown in Figure 54;
Figure 56 is a left side view of the latch jaw to be mounted on the latch head shown in Figures 49 to 52;
Figure 57 is a bottom view of the latch jaw shown in Figure 56;
Figure 58 is a rear view of the latch jaw shown in Figures 56 and 57;
Figure 59 is a left side view of the jaws assembly in the open position, the jaws assembly being made up of the latch head shown in Figures 49 to 52, the spring retainer shown in Figures 54 and 55, the latch jaw shown in Figures 56 to 58, a latch spring, and pins to assemble the various components together;
Figure 60 is a bottom view of the jaws assembly shown in Figure 59, with the jaws assembly being shown in the open position;
Figure 61 is a left side view of the jaws assembly shown in Figures 59 and 60, with the jaws assembly being shown in the closed position (and in the open position in phantom lines);
Figure 62 is a bottom plan view of the main pump unit chassis;
Figure 63 is a front view of the main pump unit chassis shown in Figure 62;
Figure 64 is a top plan view of the main pump unit chassis shown in Figures 62 and 63;
Figure 65 is a rear view (inverted) of the main pump unit chassis shown in Figures 62 to 64;
Figure 66 is a perspective top view of the cassette guide used to position the cassette of Figures 45 to 48 on the main pump unit;
Figure 67 is a sectional view of the cassette guide shown in Figure 66;
Figure 68 is a top view of the cassette guide shown in Figures 66 and 67;
Figure 69 is a bottom view of the cassette guide shown in Figures 66 to 68;
Figure 70 is a side view of the pump shaft on which the jaws assembly shown in Figures 59 to 61 is mounted;
Figure 71 is a right side view of the slide lock used to retain the cassette shown in Figures 43 to 48 in position on the main pump unit;
Figure 72 is a bottom view of the slide lock shown in Figure 71;
Figure 73 is a left side view (inverted) of the slide lock shown in Figures 71 and 72, showing the bevel used to reflect the light beam from the optical light source away from the optical light sensor when the slide lock is in the open position;
Figure 74 is a top view of the slide lock shown in Figures 71 to 73, showing the reflective surface used to reflect the light beam from the optical light source to the optical light sensor when the slide lock is in the closed position;
Figure 75 is a front view of the slide lock shown in Figures 71 to 74;
Figure 76 is a rear view of the slide lock shown in Figures 71 to 75, showing the slanted surfaced used to reflect the light beam away from the corresponding sensor when the slide lock is in the open position;
Figure 77 is a perspective view from above of the upper sensor housing;
Figure 78 is a vertical section through the upper sensor housing shown in Figure 77;
Figure 79 is a top plan view of the upper sensor housing shown in Figures 77 and 78;
Figure 80 is a bottom plan view of the upper sensor housing shown in Figures 77 to 79;
Figure 81 is a perspective view from above of the lower sensor housing;
Figure 82 is a vertical section through the lower sensor housing shown in Figure 81;
Figure 83 is a perspective view from beneath of the lower sensor housing shown in Figures 81 and 82;
Figure 83A is a bottom plan view of the lower sensor housing shown in Figures 81 to 83;
Figure 84 is a top plan view of a portion of a flex circuit used to interface electrically with a pair of ultrasonic transducers;
Figure 85 is a partially exploded perspective view showing how the ultrasonic transducers are attached to the flex circuit using conductive transfer tape;
Figure 85A is a partially exploded perspective view showing an alternative embodiment in which portions of the flex circuit and the conductive transfer tape on the rear sides of the ultrasonic transducers have apertures therethrough;
Figure 86 is a perspective view from beneath showing the assembly of Figure 85 installed in the upper sensor housing;
Figure 87 is a perspective view from beneath showng a miniature circuit board installed on the flex circuit of the assembly of Figure 86;
Figure 88 is a front elevation of an optical sensor module;
Figure 89 is a side elevation of the optical sensor module shown in Figure 88;
Figure 90 is a top plan view of the optical sensor module shown in Figures 88 and 89;
Figure 91 is a front view of a valve actuator;
Figure 92 is a side view of the valve actuator shown in Figure 91;
Figure 93 is a bottom plan view of the valve actuator shown in Figures 91 and 92;
Figure 94 is a top plan view of one of the actuator guides used to guide and retain in position the valve actuators for one cassette;
Figure 95 is a side view of the actuator guide shown in Figure 94;
Figure 96 is a top plan view of a pressure transducer;
Figure 97 is a side view of the pressure transducer shown in Figure 96;
Figure 98 is a bottom plan view of the pressure transducer shown in Figure 96;
Figure 99 is a bottom plan view of the elastomeric valve actuator seal used to bias the valve actuators into an upward position;
Figure 100 is a cutaway view of the valve actuator seal shown in Figure 99;
Figure 101 is a perspective view of the main pump unit chassis having the various components for one pump mounted thereon;
Figure 102 is a bottom view of the main pump unit chassis having the various components for one pump mounted thereon, with the slide lock in the open position ready to receive a cassette;
Figure 103 is a bottom view of the main pump unit chassis shown in Figure 102, with the slide lock in the closed position as it would be if a cassette were installed and latched onto the main pump unit;
Figure 104 is a side view showing a cassette in position to be installed on the main pump unit;
Figure 105 is a side view showing the cassette as it is engaging the main pump unit, with the tubing adaptor engaging the flared recess in the bottom of the sensor housing to draw the outlet tube into engagement between the ultrasonic transducers;
Figure 106 is a side view showing the cassette fully installed on the main pump unit with the slide latch closed and the outlet tube in full engagement between the ultrasonic transducers in the sensor housing;
Figure 107 is a block diagram of the entire operating system of the infusion pump of the present invention, showing the ultrasonic air-in-line detector system and self test therefor;
Figure 108 is a schematic diagram of the transmitting circuitry for the ultrasonic air-in-line detector system for all three channels;
Figure 109 is a block diagram of the receiver circuitry for one channel, the circuitry having an output signal;
Figure 110 is a schematic diagram of the processing circuitry used to process the output signal from the receiver circuitry to produce an AILD Output signal for each channel and an interrupt signal indicating a change in state of the AILD Output signal of one of the three channels;
Fig. 111 shows various waveforms generated by the circuitry of Figures 108, 109, 110;
Figure 112 is a simplified flow diagram illustrating the operation of the air-in-line detector monitoring system; and
Fig. 113 is a simplified flow diagram illustrating the operation of the air-in-line detector self test system.

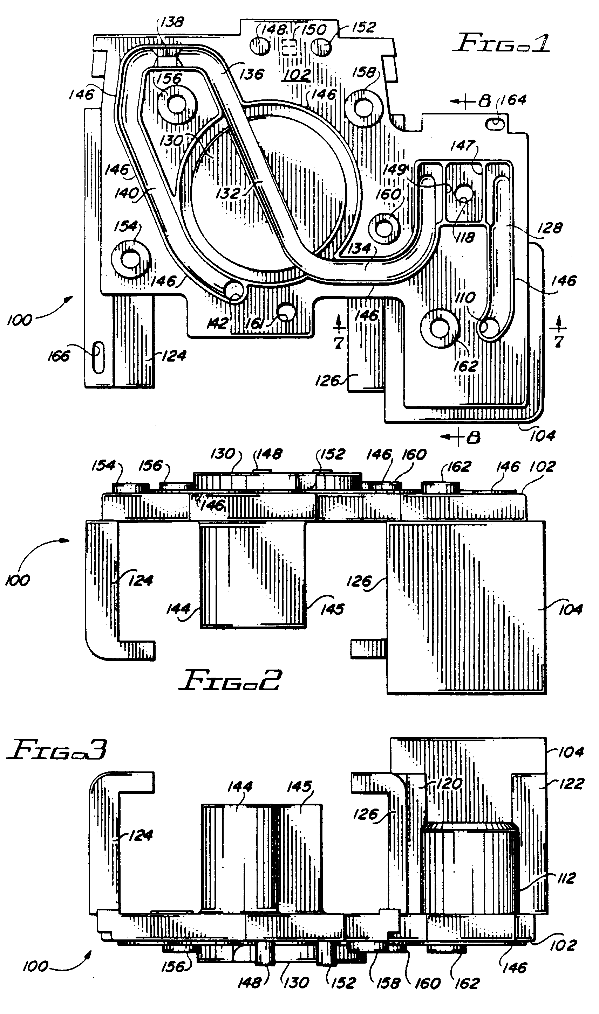

The Cassette The preferred embodiment of the cassette of the present invention includes all of the features described above in a single compact disposable cassette constructed of seven parts. The basic construction and operation of the cassette is the subject of EP-A-319278 in the name of the present Applicants, entitled "Disposable Cassette for a Medication Infusion System".

Referring next to Figures 43 and 44, a tubing adaptor 301 is shown located between an outlet tubing 306 extending from an assembled cassette 302 and a delivery tubing 303 which leads to the patient. The tubing adaptor 301 is essentially cylindrical, and is hollow throughout allowing the outlet tubing 306 and the delivery tubing 303 to be inserted into it. The inlet tubing 306 and the delivery tubing 303 in the preferred embodiment are adhesively secured in the tubing adaptor 301. Located at the top end of the tubing adaptor 301 there is an outer tapered portion 305 having a smaller outer diameter as it approaches the top end. Located below the tapered portion 305 is a radially outwardly extending flange 307.

The assembly and configuration of the cassette will now be described with reference to the assembled cassette 302 shown in Figures 45 to 48.

An inlet tube 304 is adhesively secured to the inner diameter of an inlet aperture 238 in the bubble chamber cap 230, in fluid communication with a bubble chamber 106. An outlet tube 306, extending through the wider portion of the aperture 258 and is adhesively secured to the inner diameter of the outlet tube mounting cylinder 144 in the cassette body 100.

The tubing adaptor 301 is connected to the other end of the outlet tube 306 and the delivery tube 303 is also attached to the tubing adaptor 301. The inlet tube 304 and the outlet tube 306 are shown in the figures only in part; on their respective ends not connected to the assembled cassette 302 they may have connector fittings such as standard luer connectors (not shown), which are well known in the art. The adhesives used to attach the inlet tube 304 and the outlet tube 306 to the assembled cassette 302 also represent technology well known in the art. For example, adhesives such as cyclohexanone, methylene dichloride, or tetrahydrofuron (THF) may be used.

The Main Pump Unit The preferred embodiment of the main pump unit of the present invention includes a number of components used to hold, latch, and drive the cassette 302 described above.

In Figures 62 to 65, a main pump unit chassis 370 is illustrated which is designed to mount three independent pump units including three drive mechanisms into which three disposable assembled cassettes 302 may be installed. The assembled cassettes 302 are mounted on the bottom side of the pump chassis 370 shown in Figure 62, with the motors and drive train being installed mounted on top of the pump chassis 370 (Figure 64) within a housing (not shown).

Located on the pump chassis 370 are three pairs of angled segment 372 and 374, 376 and 378 and 380 and 382. Each pair of angled segments 372/374, 376/378, 380/382 defines two facing channels between them. In the preferred embodiment, the angled segments are angled slightly further from the bottom of the pump chassis 370 near the front, to have a camming effect as an assembled cassette 302 is installed and the slide latch 240 is closed. Specifically, the angled segment 372 defines a channel facing the angled segment 374 and the angled segment 374 defines a channel facing the angled segment 372. The angled segment 376 defines a channel facing the angled segment 378, and the angled segment 378 defines a channel facing the angled segment 376. Finally, the angled segment 380 defines a channel facing the angled segment 382 and the angled segment 382 defines a channel facing the angled segment 380.

Each pair of angled segments 372/374, 376/378, 380/382, provides means on the bottom of pump chassis 370 for one assembled cassette 302 to be securely latched to. The inverted L-shaped portions 250 and 252 in the slide latch 240 (Figures 29 and 30) of the assembled cassette 302 can be attached to one of the parts of angled segments 372/374, 376/378 and 380/382. With the slide latch 240 pulled back away from the front of the assembled cassette 302, an area between the front portion 242 of the slide latch 240, and the top front of the cassette body 100 and retainer cap 190 is opened, allowing the top of the assembled cassette 302 to be placed over one of the pairs of angled segments 372/374, 376/378, 380/382.

By way of example, it will be assumed that the assembled cassette 302 is to be mounted in the first position ( the position on the left end of the pump chassis 370) on the first pair of angled segments 372/374. The top surface of the assembled cassette 302, which is the retainer cap 190 (Figure 43), will come into contact with the bottom of the pump chassis 370 (Figure 62). In order to allow the assembled cassette 302 to be installed, the slide latch 240 is pulled back fully from the front of the assembled cassette 302, leaving the open area between the front portion 242 of the slide latch 240 and the front top portion of the assembled cassette 302 (made up of the cassette body 100 and the retainer cap 190) facing the front portion 242 of the slide latch 240.

The top of the assembled cassette 302 is then placed against the bottom of the pump chassis 370 with the first pair of angled segments 372/374 fitting in the area between the front portion 242 of the slide latch 240 and the front top portion of the assembled cassette 302. The slide latch 240 is then pushed forward into the cassette body 100, sliding the inverted L-shaped portion 250 of the slide latch 240 into engagement with the angled segment 372, and sliding the inverted, backwards L-shaped potion 252 of the slide latch 240 into engagement with the angled segment 374. The assembled cassette 302 will thus be held in position on the bottom of the pump chassis 370 until the slide latch 240 is again pulled back, releasing the assembled cassette 302.

Projecting from the bottom of the pump chassis 370 are a number of components used to position and align the assembled cassettes 302 in the first (left hand end of the pump chassis 370), second (intermediate), and third (right hand end) positions on the pump chassis 370. Three left lateral support walls 384, 386 and 388 protrude from the bottom of the pump chassis 370 at locations to support the rear upper left side portion of assembled cassettes 302 in proper positions in the first, second and third positions, respectively. Likewise, three right lateral support walls 390, 392 and 394 protrude from the bottom of the pump chassis 370 at locations to support the rear upper right portion of assembled cassettes 302 in proper positions in the first, second, and third positions, respectively.

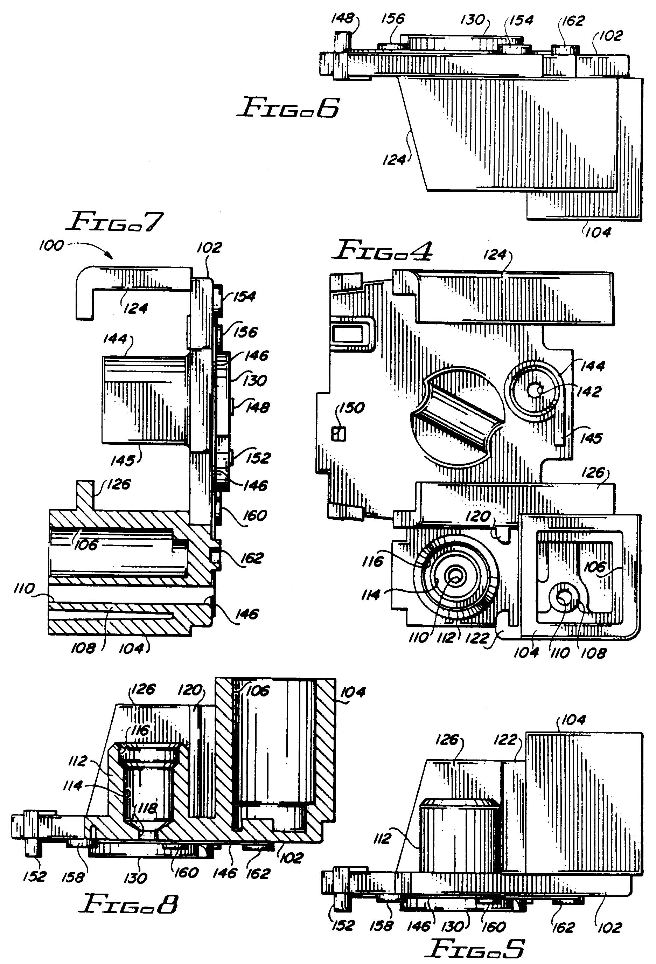

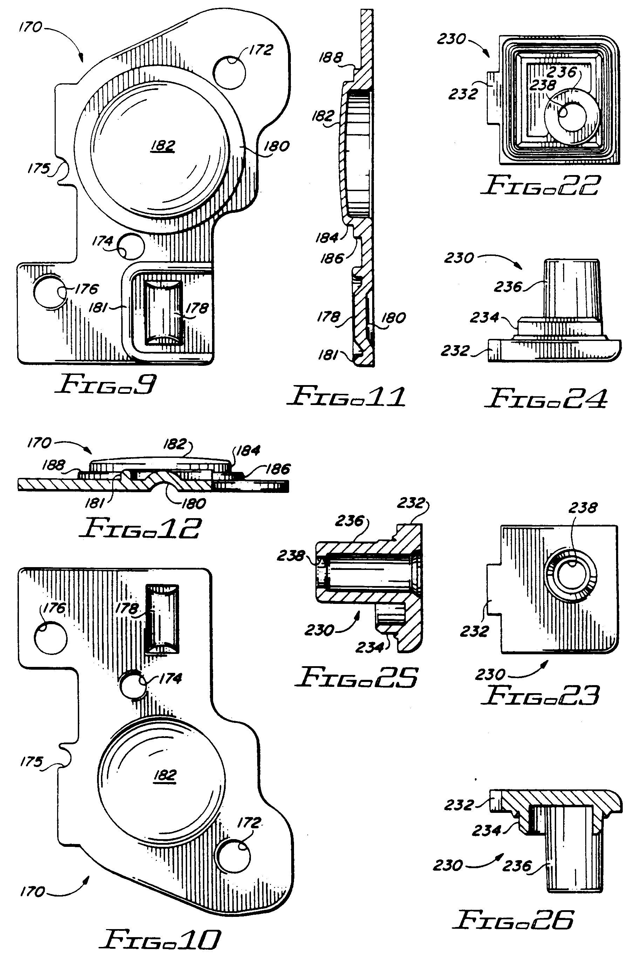

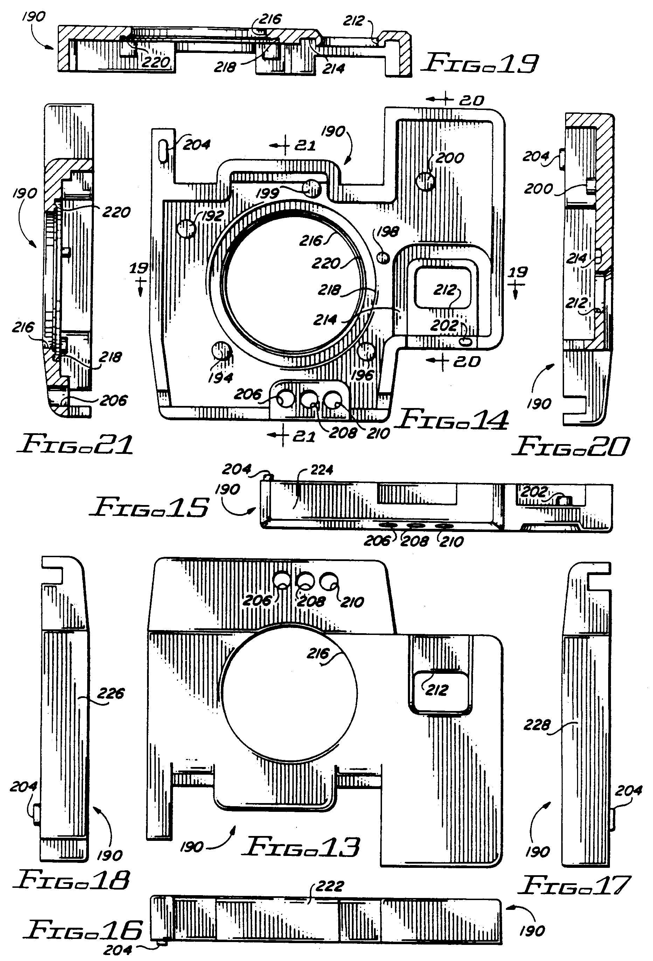

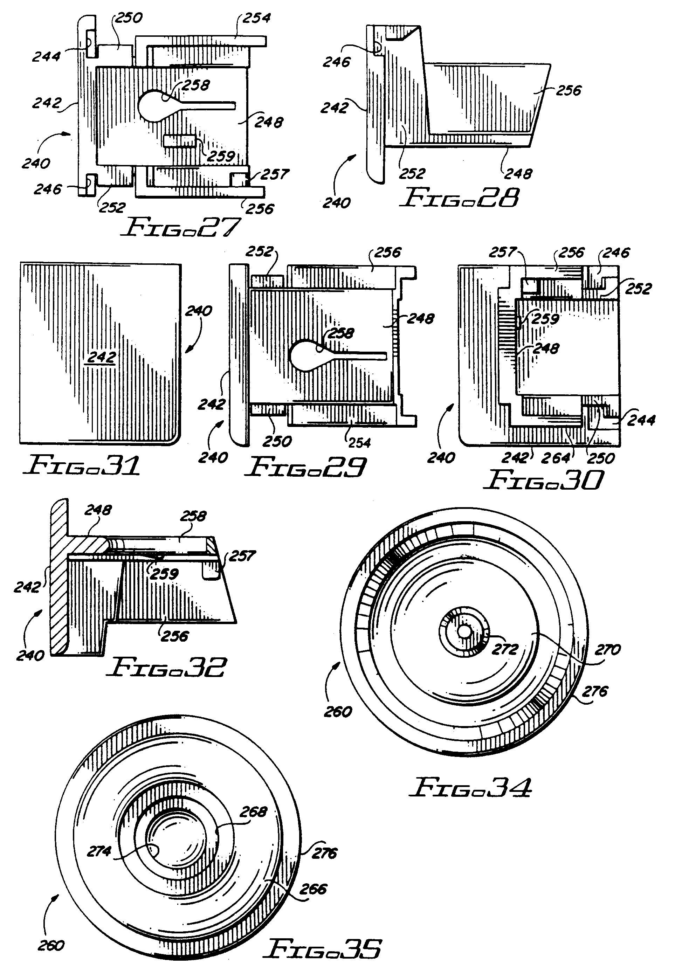

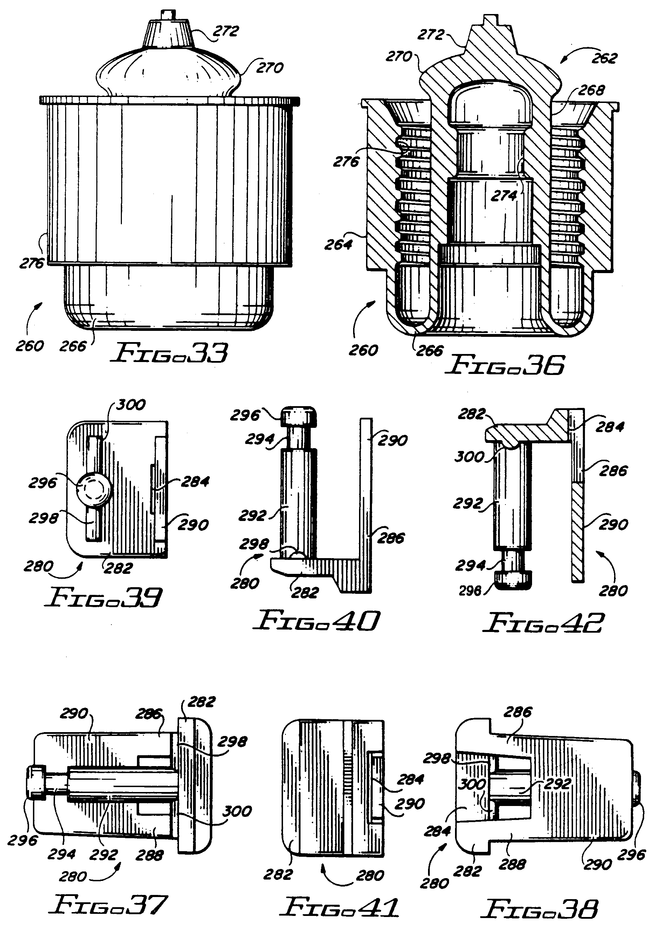

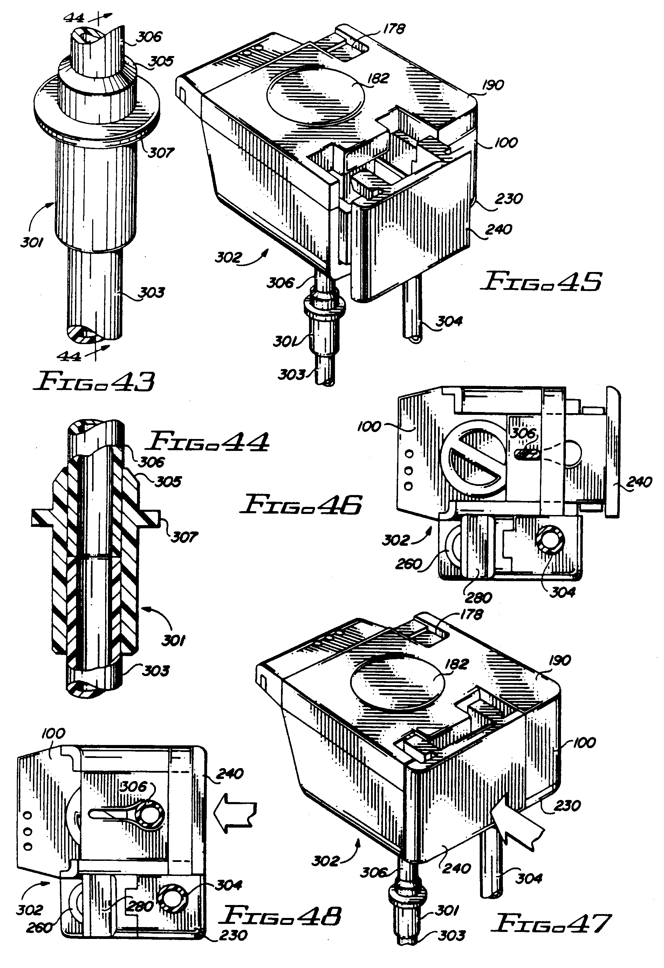

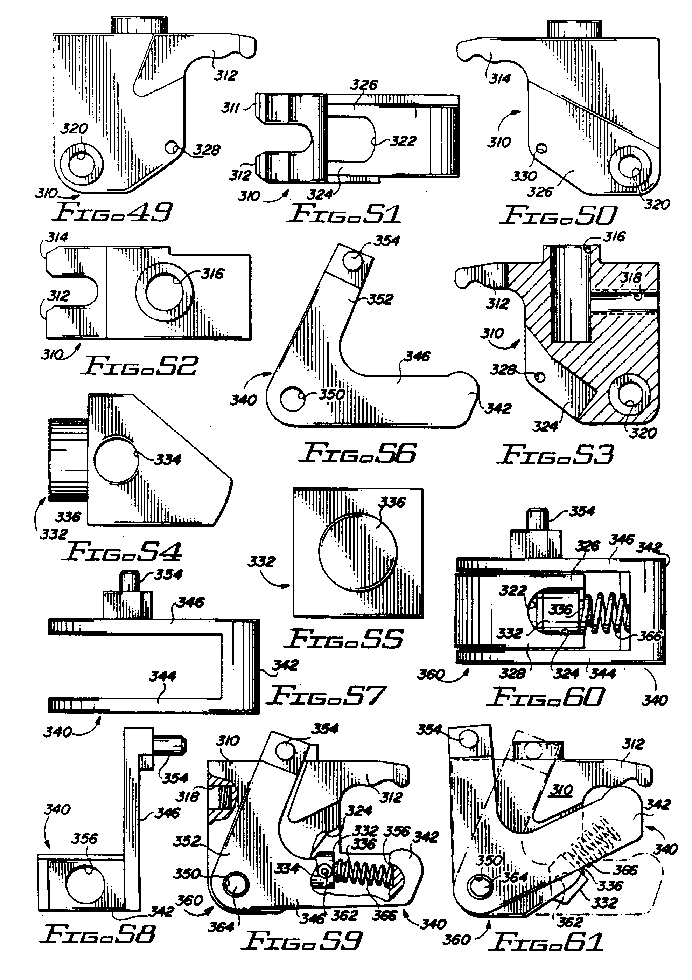

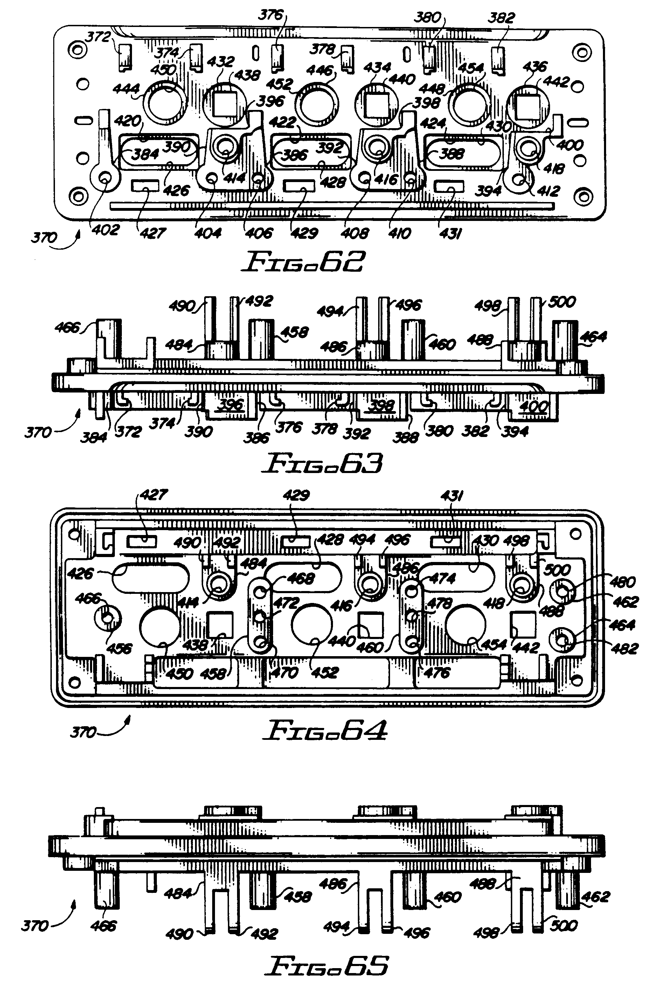

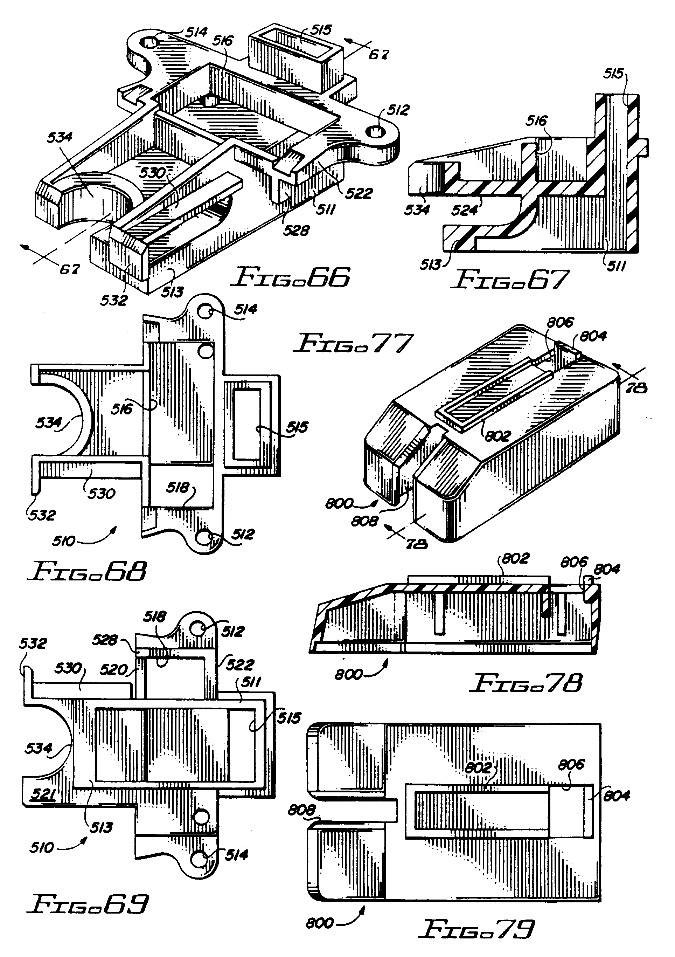

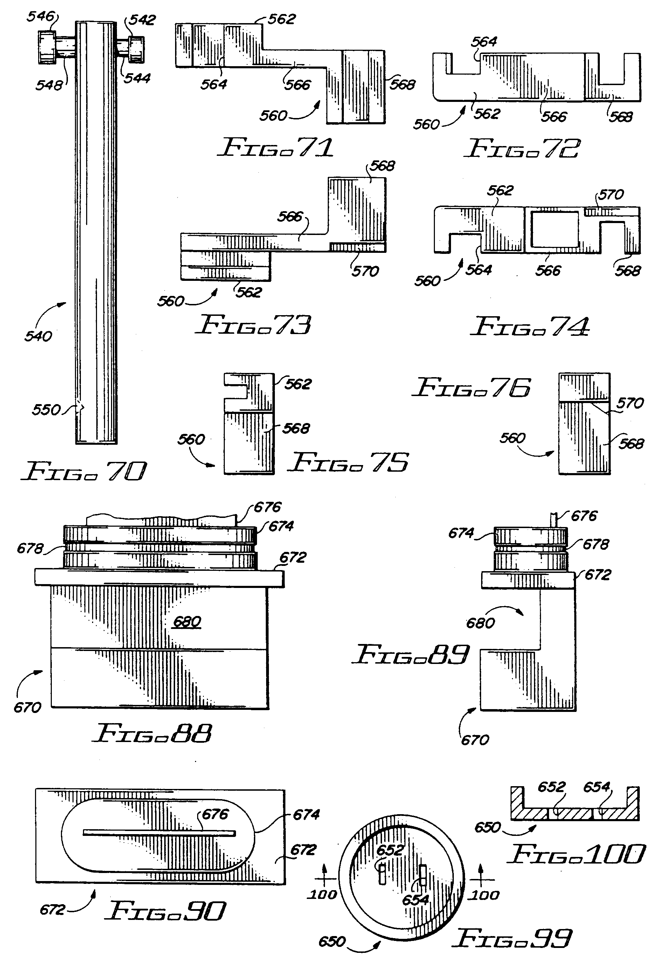

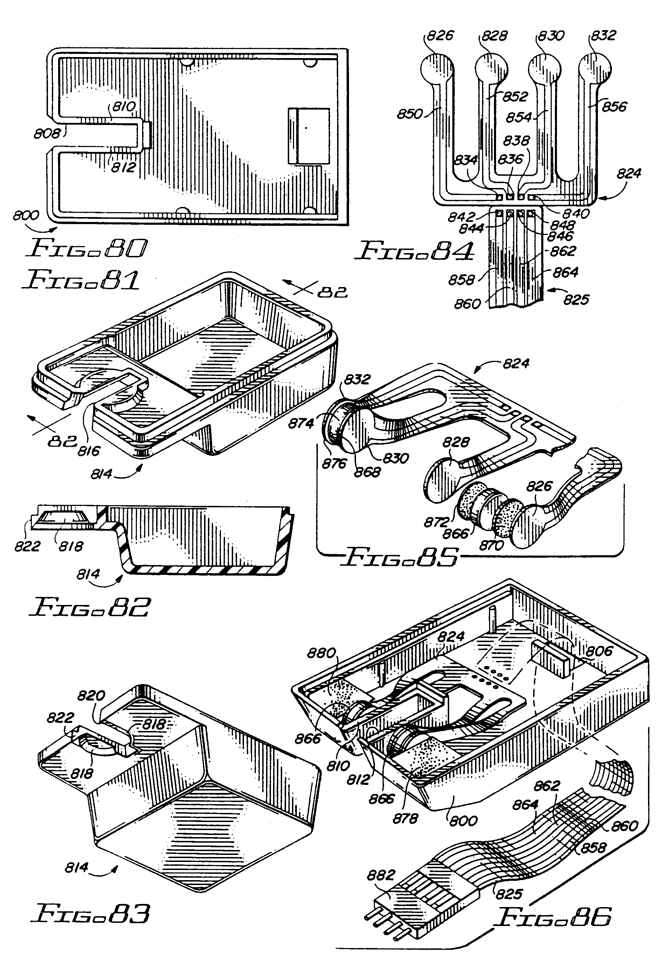

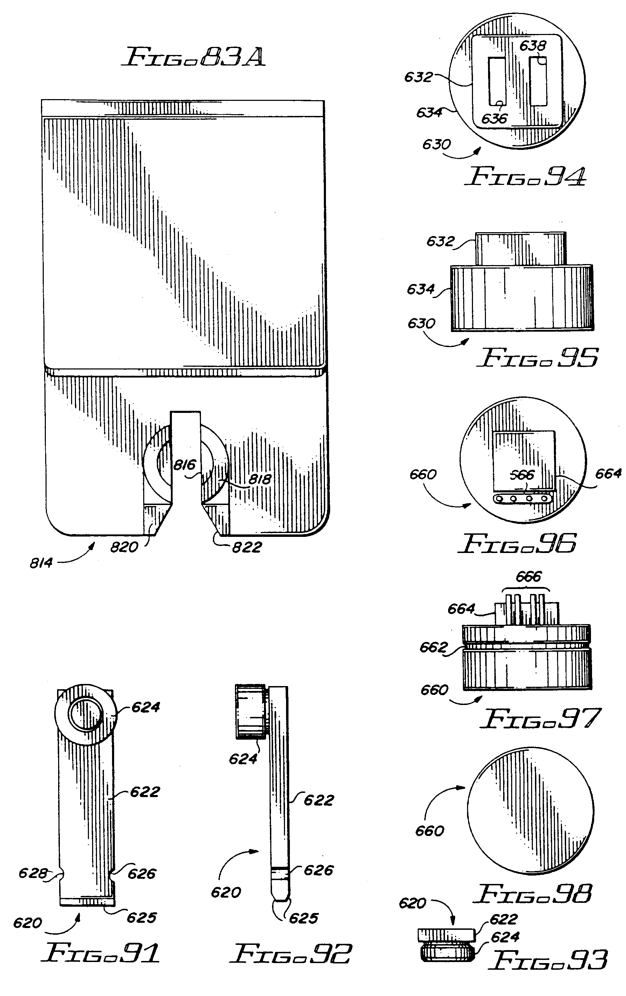

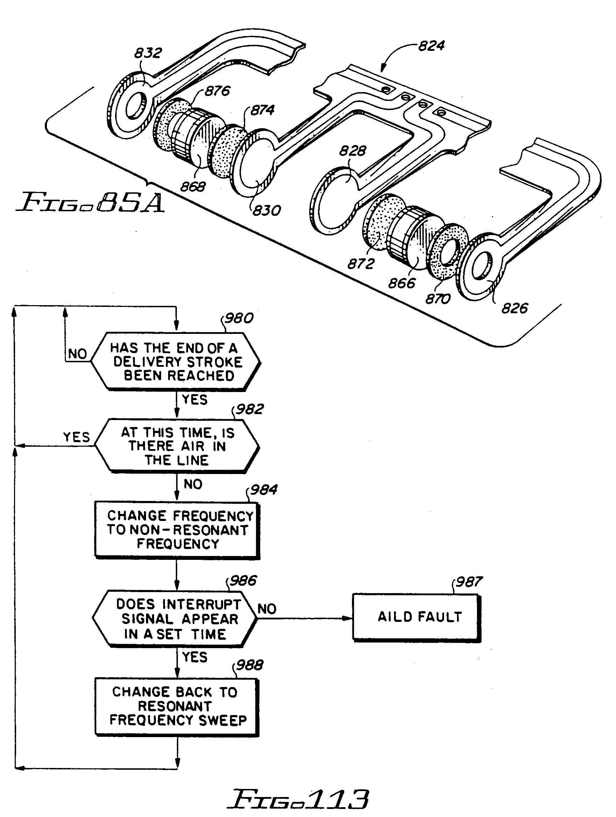

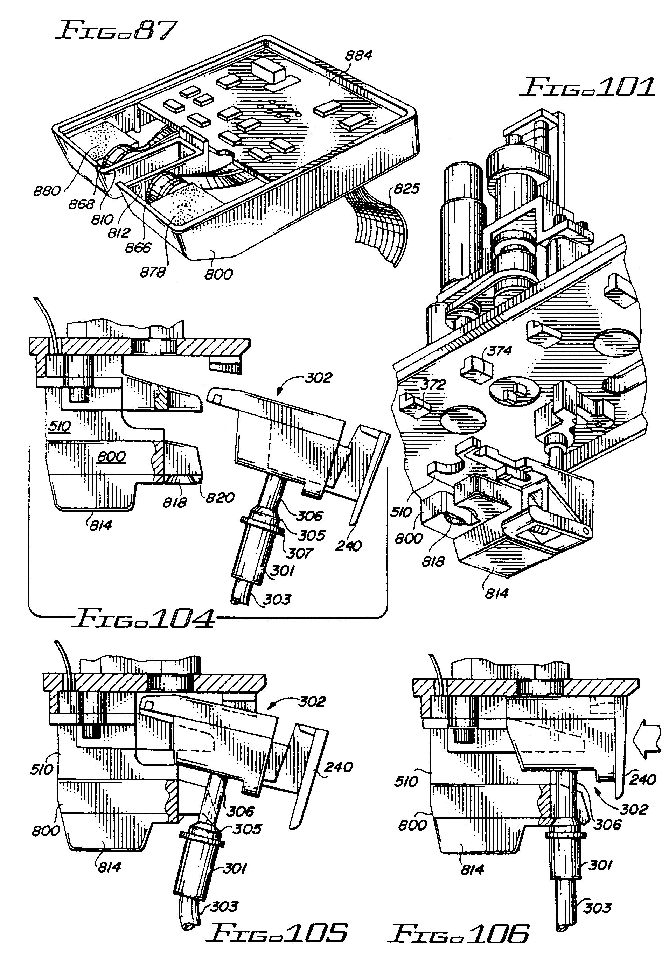

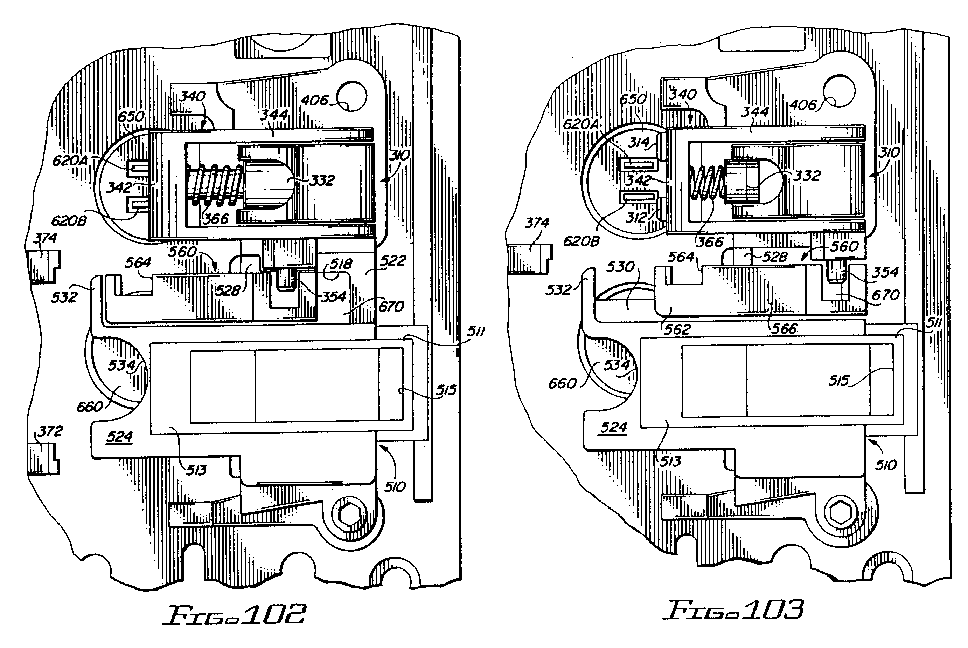

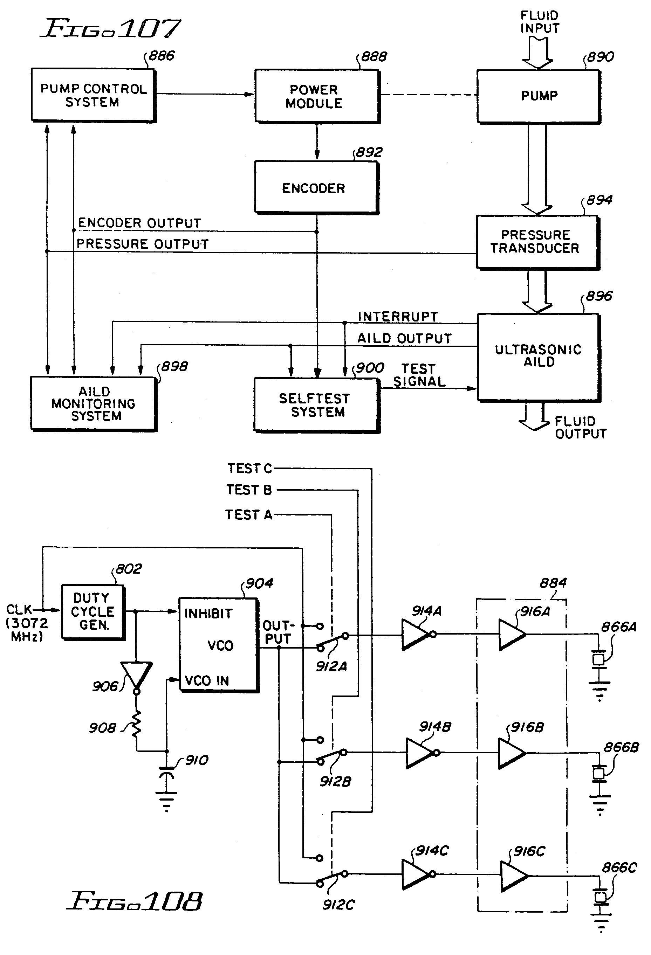

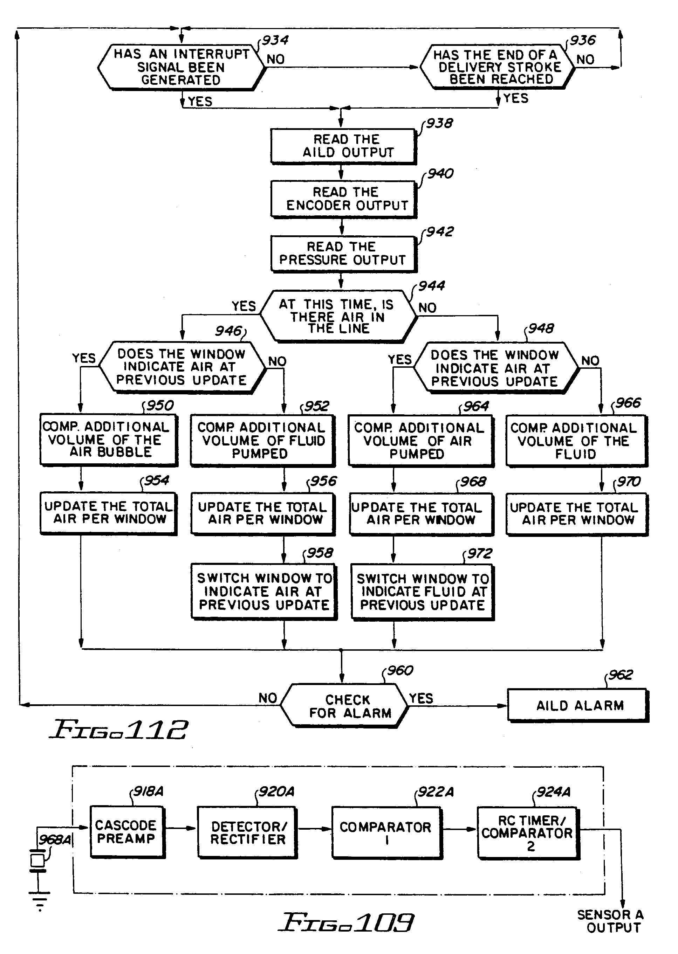

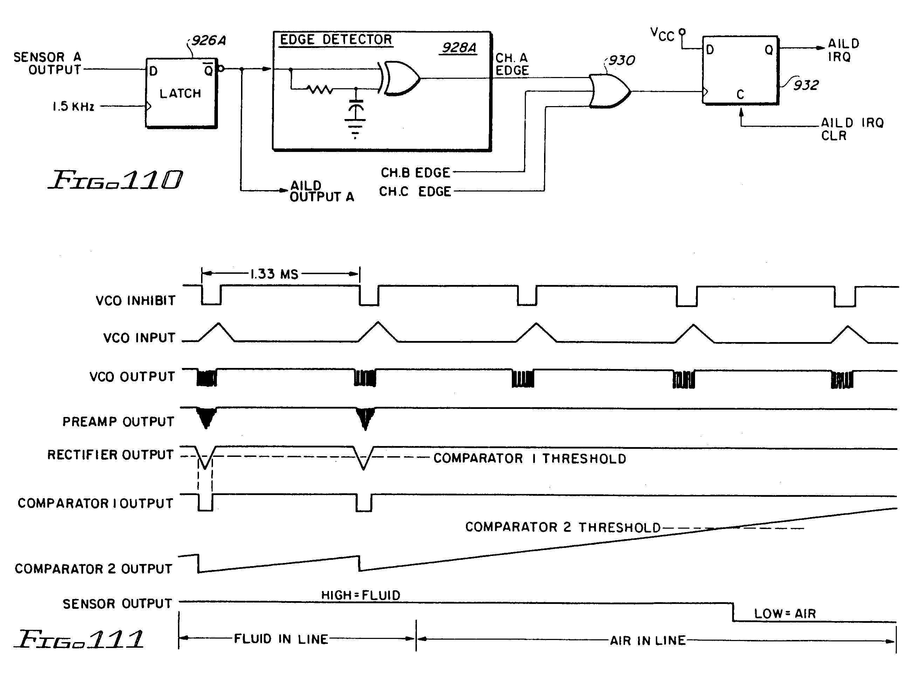

Additional support and positioning for the installation of the assembled cassettes 302 into the first, second and third positions are provided for the upper right rear corner of the assembled cassettes 302 by three right corner support walls 396, 398, and 400 respectively. The three right corner support walls 396, 398, 400 are L-shaped when viewed from beneath (Figure 62), and support and position the rear of the assembled cassettes 302 behind the pump cylinders 112 (Figure 4) and a portion of the right side of the assembled cassettes 302 adjacent the pump cylinders 112. Note that the three right lateral support walls 390, 392, and 394 and the three right corner support walls 396, 398 and 400 together provide continuous support and positioning for the assembled cassettes 302 in the first, second and third positions, respectively.

A threaded aperture 402 is formed in the raised material forming the left lateral support wall 384, near the rear. A single segment of raised material forms the right lateral support wall 390, the right corner support wall 396, and the left lateral support wall 386. Formed in that segment of raised material near the rear is a threaded aperture 404 on the left side near the right lateral support wall 390, and a threaded aperture 406 on the right side near the left lateral support wall 386. Similarly, a single segment of raised material forms the right lateral support wall 392, the right corner support wall 398, and the left lateral support wall 388, and includes corresponding apertures 408 and 410 on the left and right respectively. Finally, a single segment of raised material forms the right lateral support wall 394 and the right corner support wall 400 and includes a threaded aperture 412 at the rear near the right lateral support wall 394.

In the segment of raised material forming the support walls 390, 396 and 386, near the former where the right lateral support wall 390 and the right corner support wall 396 meet, there is an aperture 414 which extends through the thickness of the pump chassis 370. A similar aperture 416 is formed in the segment of raised material forming the support walls 392, and 388. In the segment of raised material forming he right lateral and corner support walls 394 and 400 near the corner where these walls meet, there is an aperture 418 which extends through the thickness of the pump chassis 370.

When an assembled cassette 302 is positioned and mounted in the first, second, and third positions, the apertures 414, 416 and 418 respectively, will coincide directly with the piston rods 292 of the assembled cassettes 302 (Figure 46). The apertures 414, 416 and 418 will be used to mount the drive shafts connected to the jaws assembles 360 (Figures 59 to 61) used to drive the piston assembly 280.

Located between the left lateral support wall 384 and the right lateral support wall 390, there is a longitudinal rectangular recess 420 in the bottom surface of the pump chassis 370. Similarly, located between the left lateral support wall 386 and the right lateral support wall 392, there is a longitudinal rectangular recess 422 and located between the left. lateral support wall 384 and the right lateral support wall 390, there is a longitudinal rectangular recess 424. While the rectangular recesses 420, 422, 424, do not extend through the pump chassis 370, oval apertures 426, 428, and 430 smaller than the rectangular recesses 420, 422, 424, are located in the rectangular recesses 420, 422, and 424 respectively, and extend through to the top side of the pump chassis 370. The rectangular recess 420, 422 and 424 will house sensor modules, and the oval aperture 426, 428 and 430 allow wires from the sensor modules to extend through the pump chassis 370. With the assembled cassettes 302 positioned and mounted in the fist, second, and third positions, the rear-most parts of the upper portions of the assembled cassettes 302 will be located beneath the rectangular recesses 420, 422 and 424. Located behind the oval aperture 426, 428, and 430 are rectangular apertures 427, 429, and 431, respectively. The rectangular apertures 427, 429, and 431 are to allow the wires from the ultrasonic sensors to extend through the pump chassis 370.

Located in front of the right corner support wall 396 is a circular recess 432 in the bottom surface of the pump chassis 370.

Similar circular recesses 434 and 436 are formed in the bottom surface of the pump chassis 370 in front of the right corner support walls 398 and 400. While the circular recesses 432, 434, and 436 do not extend through the pump chassis 370, square apertures 438, 440 and 442, smaller than the circular recesses 432, 434, and 436, are located in the circular recesses 432, 434, and 436 respectively and extend through the top side of the pump chassis 370.

The circular recesses 432, 434, and 436 will be used to house valve actuator guides, and the square apertures 438, 440 and 442 allow valve actuators to extend through the pump chassis 370 and to orient the valve actuator guides. With the assembled cassettes 302 positioned and mounted in the first, second and third positions, the circular recesses 432, 434, and 436, respectively, will correspond exactly with the locations of the domed portions 178 of the valve diaphragms 170 in the assembled cassettes 302 (Figure 43).

Located to the left of the circular recess 432 and in front of the rectangular recess 420, there is a circular recess 444 in the bottom surface of the pump chassis 370. Similarly, located to the left of the circular recess 434 and in front of the rectangular recess 422, there is a circular recess 446, and located to the left of the circular recess 436 and in front of the rectangular recess 424, there is a circular recess 448. While the circular recesses 444, 446, 448 do not extend through the pump chassis 370, cylindrical apertures 450, 452 and 454 of a smaller diameter than the circular recesses 444, 446 and 448 are located in the circular recesses 444, 446 and 448 respectively, and extend through to the top side of the pump chassis 370.

The circular recesses 444, 446 and 448 will be used to house pressure transducers and the cylindrical apertures 450, 452, and 454 allow wires from the pressure transducers to extend through the pump chassis 370. With the assembled cassettes 302 positioned and mounted in the first, second, and third positions, the circular recesses 444, 446 and 448 respectively, will correspond with the locations of the pressure diaphragms 182 of the valve diaphragms 170 in the assembled cassettes 302 (Figure 43).

Projecting from the top surface of the pump chassis 370 are a number of raised elements in which threaded apertures are located to support the drive assembly. A cylindrical raised segment 456 is located to the left of the cylindrical aperture 450, a laterally extending generally oval raised segment 458 is located between the square aperture 438 and the cylindrical aperture 452, and a second laterally extending generally oval raised segment 460 is located between the square aperture 440 and the cylindrical aperture 454. In addition, two cylindrical raised segments 462 and 464 are located to the right of the square aperture 442 laterally aligned with the rear-most and front-most portions respectively of the oval raised segments 458 and 460.

A threaded aperture 466 is formed in the cylindrical raised segment 456. Located in the oval raised segment 458, there is a threaded aperture 468 near the rear, a threaded aperture 470 near the front, and a central threaded aperture 472. Similarly, the oval raised segment 460 includes a threaded aperture 474 near the ear, a threaded aperture 476 near the front, and a central threaded aperture 478. The cylindrical raised segments 462 and 464 include threaded apertures 480 and 482 respectively.

The apertures 414 and 416 and 418 through the pump chassis 370 terminate in raised segments 484, 486 and 488 respectively extending from the top surface of the pump chassis 370 and surrounding the respective apertures. Extending upwards from the raised segment 484 behind the aperture 414 on the left side is a guide finger 490, and on the right side is a guide finger 492. The guide fingers 490 and 492 are parallel and have a space between them. Similar pairs of guide fingers 494/496 and 498/500 extend upwardly from the raised segment 486 behind the aperture 416 and from the raised segment 488 behind the aperture 418.

Figures 66 to 69 show a cassette guide 510 for use in guiding the installation of the assembled cassette 302 into the proper location for latching on the pump chassis 370. At the rear the cassette guide 510 has an aperture 512 on the right and an aperture 514 on the left. The aperture 512 will be aligned with one of the threaded apertures 404, 408 or 412 (Figure 62) while the aperture 514 will be aligned with one of the threaded apertures 402, 406 or 410, to install the cassette guide 510 in either the first, second, or third position.

The top side (Figure 66) of the cassette guide 510 has a rectangular recess 516 which corresponds in size to the rectangular recesses 420, 422 and 424 in the pump chassis 370. The sensor modules will be accommodated between the rectangular recesses 516 in the cassette guides 510 and the rectangular recesses 420, 422, and 424 in the pump chassis 370. The right hand side of this rectangular recess 516 is exposed through a rectangular aperture 518 in the cassette guide 510 (Figure 67).

An area 520 on the bottom of the cassette guide 510 immediately in front of the rectangular aperture 518 and an area 522 immediately to the right and to the rear of the rectangular aperture 518 is recessed upwards from the under surface 524 of the cassette guide 510. At the front right corner of the rectangular aperture 518, a square segment 528 extends downwards to the level of the bottom surface 524 of the cassette guide 510. Located immediately forward of the square segment 528 is a thin rectangular track 530 extending from the right side of the cassette guide 510. The thin rectangular track 530 terminates at its front end in a blocking segment 532.

The front end of the cassette guide 510 has a rounded notch 534, which is positioned to receive the outlet tube mounting cylinder 144 on the cassette body 100 (Figure 4) when the cassette guide 510 is installed on the pump chassis 370. When the cassette guide 510 in installed on the pump chassis 370, the rear-most portion of the assembled cassette 302 will fit between the cassette guide 510 and the bottom of the pump chassis 370. Accordingly, the cassette guide 510, together with the various support walls on the bottom of the pump chassis 370, aids in the installation of the assembled cassettes 302 in the proper position for latching.

Extending downwards from the surface 524 is a hollow lower segment 511 having a projection 513 extending towards the front. When the assembled cassette 302 is installed, the horizontal bottom portion 248 of the slide latch 240 will be located between the surface 524 and the projection 513. The lower segment 511 is hollow in order to receive the ultrasonic sensor housing, as will become apparent below. A hollow chimney 515 is located at the back of the cassette guide 510, and is in communication with the interior of the lower segment 511. When the cassette guide 510 is installed on the pump chassis 370, the interior of the hollow chimney 515 will be in communication with one of the rectangular apertures 427, 429, 431 to allow wires from the ultrasonic sensor to extend therethrough.

The power module to drive the main pump unit will not be described in detail since it is not closely related to the subject matter of the present invention. For a complete description of the construction of the power module, EP-A-319277, referred to above and incorporated herein by reference may be referred to.

Figures 77 to 80 show an upper ultrasonic housing 800. The upper ultrasonic housing 800 is hollow, and is open at the bottom. The upper surface of the upper ultrasonic housing 800 has a U-shaped ridge 802 and a straight ridge 804, with a rectangular aperture 806 located between them in the upper surface. The U-shaped ridge 802 and the straight ridge 804 are sized to fit within the lower segment 511 of the cassette guide 510 (Figure 69).

Located in the front of the upper ultrasonic housing 800, there is a slot 808 for receiving the outlet tube 306 of the assembled cassette 302. The slot 808 is deeper than it is wide, and has a funnel-shaped entrance to allow the outlet tube 306 to be directed easily into the slot 808. In the preferred embodiment, the width of the slot 808 is narrower than the outside diameter of the outlet tube 306, so that when the outlet tube 306 fits in the slot 80, it is deformed somewhat.

The interior of the upper ultrasonic housing 800 may be thought of as three areas, one on each side of the slot 808, and a third area representing that portion of the upper ultrasonic housing 800 into which the slot 808 does not extend. The first two areas are locations in which ultrasonic transducers (not shown) will be located, and the third area will be the location of a miniature printed circuit board (not shown). Referring particularly to Figure 80, the first area, in the front and on the right-hand side of the upper ultrasonic housing 800, is bounded by a wall 810 on the right of the slot 808. The second area, in the front and on the left-hand side of the upper ultrasonic housing 800, is bounded by a wall 812 on the left of the slot 808.

Figures 81 to 83 show a lower ultrasonic housing 814 which will be mounted onto the bottom of the upper ultrasonic housing 800. Like the upper ultrasonic housing 800, the lower ultrasonic housing 814 is hollow, but the lower ultrasonic housing 814 is open at the top. The front portion of the lower ultrasonic housing 814 (the portion which will be under the first two areas inside the upper ultrasonic housing 800) is shallow, while the rear portion of the lower ultrasonic housing 814 is deeper. The lower ultrasonic housing 814 also has a slot 816 which will be located under the slot 808 in the upper ultrasonic housing 800 when the lower ultrasonic housing 814 is mounted on the upper ultrasonic housing 800. The slot 816 also has a funnel-shaped entrance, like the slot 808.

Beneath the portion of the lower ultrasonic housing 814 having the slot 816, there is a recessed area 818. The recessed area 818 is located on both the left and the right-hand sides of the slot 816. In the preferred embodiment, the recessed area 818 is frustroconically shaped, as best shown in Figures 83 and 83A. The frustroconically shaped recessed area 818 is spaced slightly away from the front of the lower ultrasonic housing 814. Located on the bottom and at the front of the lower ultrasonic housing 814 on each side of the slot 816 there are two ramps 820 and 822 which are inclined towards the frustroconically shaped recessed area 818.

The recessed area 818 and the two ramps 820 and 822 are designed to capture and retain the tapered portion 305 of the tubing adaptor 301 (Figure 43). Accordingly, the size of the recessed area 818 is substantially the same as the size of the tapered portion 305 of the tubing adaptor 301. The two ramps 820 and 822 are located as shown in Figure 83A to draw the tapered portion 305 of the tubing adaptor 301 from a position on the two ramps 820 and 822 to a position in contact with the recessed area 818. This operation of engagement of the tapered portion 305 of the tubing adaptor 301 with the recessed area 818 will be discussed further in detail below.

Figure 84 shows a portion of a two-piece flex circuit 824 and 825. The flex circuit 824 may be thought of as a straight base portion having four orthogonally extending side arms. An exposed circular conductive pad 826, 828, 830, 832 is located at the end of each of the four arms. A series of four terminals 834, 836, 838, 840 are located on the flex circuit 824 on the base portion near its centre. The conductive pads 826, 828, 830, 832 are electrically connected to the terminals 834, 836, 838, 840 by conductors 850, 852, 854, 856 respectively.

The flex circuit 825 is a long tail segment having four terminals 842, 844, 846 and 848 on the end adjacent the flex circuit 824. The base portion of the flex circuit 824 and the flex circuit 825 are to be located close together, and thus effectively form a "T". Four more conductors 858, 860, 862 and 864 are located in the flex circuit 825. The conductors 858, 860, 862, 864 are electrically connected to the terminals 842, 844, 846, 848, respectively. It will be appreciated by those skilled in the art that the conductors 850, 852, 854, and 856 and the conductors 858, 860, 862 and 864 are electrically insulated on both sides.

Figures 85 shows the assembly of two ultrasonic transducers 866 and 868 to the flex circuit 824. The transducers 866 and 868 are typically ceramic ultrasonic transducers. In a typical known assembly of ultrasonic transducers, soldering is used, with the danger of possible damage to the ceramic ultrasonic transducer. The present invention instead uses conductive adhesive transfer tape, which has adhesive on both sides and is electrically conductive. Such conductive transfer tape is commercially available from 3M under the product identification number 9703. A disc-shaped segment of conductive transfer tape 870 both secures the conductive pad 826 to the one side of the ultrasonic transducer 866 and makes electrical contact between the conductive pad 826 and the one side of the ultrasonic transducer 866.

Similarly, a disc-shaped segment of conductive transfer tape 872 is placed between the conductive pad 828 and the other side (the front side) of the ultrasonic transducer 866. A disc-shaped segment of conductive transfer tape 874 is placed between the conductive pad 830 and one side (the front side) of the ultrasonic transducer 868. A disc-shaped segment of conductive transfer tape 876 is placed between the conductive pad 832 and the other side (the back side) of the ultrasonic transducer 868. Thus, the ultrasonic transducers 866 and 868 are assembled and electrically connected to the flex circuit 824.

The disc-shaped segments of conductive transfer tape 870, 872, 874 and 876 are used in the preferred embodiment. Instead of using conductive transfer tape, conductive epoxy could be used, although the conductive transfer tape is preferred.

Referring next to Figure 86, the ultrasonic transducers 866 and 868 are assembled into the upper ultrasonic housing 800. The portion of the flex circuit 824 on the side of the conductive pad 828 opposite the ultrasonic transducer 866 is adhesively bonded to the wall 812, thus securing the ultrasonic transducer 866 to the wall 812. Similarly, the portion of the flex circuit 824 on the side of the conductive pad 830 opposite the ultrasonic transducer 868 is adhesively bonded to the wall 810, thus securing the ultrasonic transducer 868 to the wall 810. The adhesive used is preferably an elastomeric adhesive which can be applied in a thin coat with no air pockets. One such adhesive is Black Max adhesive. A small block of foam 878 is used to bear against the ultrasonic transducer 866 and the associated attached portions of the flex circuit 824. Similarly, a small block of foam 880 is used to bear against the ultrasonic transducer 868 and the associated attached portions of the flex circuit 824.

The flex circuit 825 is directed through the rectangular aperture 806 in the upper ultrasonic housing 800. The connectors 858, 860, 862 and 864 are electrically connected to a connector 882. A small printed circuit board 884 having various components thereon (Figure 87) is electrically connected to the terminals 834, 836, 838 and 840 (Figure 84) on the flex circuit 824 and the terminals 842, 844, 846 and 848 on the flex circuit 825. The printed circuit board 884 then rests in the third area in the upper ultrasonic housing 800, as shown.

In an alternative embodiment, illustrated in Figure 85A, there are apertures in the conductive pads and the disc-shaped segments of conductive transfer tape located on the back sides of each of the ultrasonic transducers 866 and 868. Thus, the conductive pad 826 and segment 870 each have an aperture extending through to the back side of the ultrasonic transducer 866. Similarly, the conductive pad 832 and the segment 876 each have an aperture extending through to the back side of the ultrasonic transducer 868. The apertures allow the ultrasonic transducers 866 and 868 to flex more freely, and the strength of the output signal is approximately doubled by using these apertures.

The apertures in the conductive pads 826 and 832 and in the disc-shaped segments of conductive transfer tape 870 and 876 are located centrally. The diameters of the ultrasonic transducers 866 and 868, and the diameters of the conductive pads 826, 828, 830 and 832, are approximately 0.21 inches (5.3mm). In the preferred embodiment, the diameters of the apertures in the conductive pads 826 and 832 and in the segments 870 and 876 are approximately 0.125 inches (3.2mm). The size of the apertures is dictated on the one hand by the desire to maintain a low resistance connection and on the other hand by the desire to maximise the amount of flexion in the ultrasonic transducers 866 and 868.

The upper ultrasonic housing 800 and its associated components as shown in Figure 87 are covered by attaching the lower ultrasonic housing 814. In the preferred embodiment, one of three manufacturing techniques may be used to attach the upper ultrasonic housing 800 and the lower ultrasonic housing 814 together. They may be adhesively secured together, they may be ultrasonically welded together, or a potting material may be used to fill the interiors of both components to produce a potted assembly. The upper ultrasonic housing 800 is then adhesively attached to the cassette guide 510, with the flex circuit 825 extending through the chimney 515 of the cassette guide 510. The U-shaped ridge 802 and the straight ridge 804 fit into the interior of the lower segment 511 of the cassette guide 510, and the adhesive securely attaches the upper ultrasonic housing 800 to the cassette guide 510.

The cassette guides 510 together with the slide locks 560 may then be mounted into the three pump positions on the pump chassis 370. In the first pump position, the flex circuit 825 which extends through the chimney 515 of the cassette guide 510 is fed through the rectangular aperture 427 in the pump chassis 370. A screw is introduced through the aperture 514 in the cassette guide 510 into the threaded aperture 402 in the pump chassis 370, and a second screw is introduced through the aperture 512 in the cassette guide 510 into the threaded aperture 404 in the pump chassis 370.

In the second pump position, the flex circuit 825 which extends through the chimney 515 of the cassette guide 510 is fed through the rectangular aperture 429 in the pump chassis 370. A screw is introduced through the aperture 514 in the cassette guide 510 into the threaded aperture 406 in the pump chassis 370, and a second screw is introduced through the aperture 512 in the cassette guide 510 into the threaded aperture 408 in the pump chassis 370. In the third pump position, the flex circuit 825 which extends through the chimney 515 of the cassette guide 510 is fed through the rectangular aperture 431 in the pump chassis 370. A screw is introduced through the aperture 514 in the cassette guide 510 into the threaded aperture 410 in the pump chassis 370, and a second screw is introduced through the aperture 512 in the cassette guide 510 into the threaded aperture 412 in the pump chassis 370. By way of example, the cassette guide 510 and the slide lock 560 are shown mounted in the first pump position in Figure 101.

The installation of the assembled cassette 302 at the first pump position, will now be described. The installation of the assembled cassette into the other two pump positions is identical to the installation into of the first pump position.

With the slide latch 240 pulled back fully away from the front of the assembled cassette 302 (Figures 45 and 46), the wider portion of the elongate, tear-shaped aperture 258 in the slide latch 240 will close the tube 306, preventing fluid from flowing though the assembled cassette 302. The inlet tube 304 is connected to a fluid source such as an IV bag (not shown), and the delivery tube 303 is connected to a fluid delivery device such as an injection set (not shown), the use of which is well known in the art. The slide latch 240 is opened, together with any other closures in the IV bag line, and fluid fills the lines, the assembled cassette 302, and the injection set. By tapping or shaking the assembled cassette 302 any residual air bubbles will flow out through the line. The slide latch 240 is then pulled back and the outlet tube 306 is closed, and the system is in a primed condition with the assembled cassette 302 ready to be installed onto the main pump unit.

When the slide latch 240 is pulled back, an opening is left between the front portion 242 of the slide latch 240 and the front top portion of the assembled cassette 302 (made up of the cassette body 100 and the retainer cap 190) facing the front portion 242 of the slide latch 240. In this example, where the assembled cassette 302 is to be mounted in the first position, the opening between the front portion 242 of the slide latch 240 and the front top portion of the assembled cassette 302 will admit the fist pair of angled segments 372 and 374 as the assembled cassette 302 is installed. The top surface of the assembled cassette 302, which is the retainer cap 190 (Figure 43), will abut against the bottom of the pump chassis 370 (Figure 62)

Prior to installing the assembled cassette 302 into the main pump unit, the slide lock 560 must be fully forward with the latch jaw 340 opened away from the latch head 310, as mentioned previously and as shown in Figure 99. In addition, the jaws assembly 360 should be in its fully upward position.

Referring now to Figure 104, the rearmost edge of the assembled cassette 302 is tilted upwards in front of the first pump position. The angled position of the tubing adaptor 301 should also be noted. The rearmost edge of the top of the assembled cassette 302 is then placed against the bottom of the pump chassis 370 between the pressure transducer 660 (mounted flush with the bottom of the pump chassis 370) and the top side of the cassette guide 510, as shown in Figure 105. As the assembled cassette 302 is so positioned, the outlet tube 306 will begin to move into the funnel-shaped entrances to the slots 808 and 816 in the upper ultrasonic housing 800 and the lower ultrasonic housing 814, respectively. Simultaneously, the top of the tapered portion 305 of the tubing adaptor 301 will contact the ramps 820 and 822 on the lower ultrasonic housing 814, as shown in Figure 105. This engagement is key, since the ramps 820 and 822 will urge the tapered portion 305 of the tubing adaptor 301 rearwards toward the recessed area 818.

The rearmost portion of the top of the assembled cassette 302 is slid towards the back of the pump chassis 370 into position between the left lateral support wall 384 on the left-hand side and the right lateral support wall 390 on the right-hand side.

As this movement of the assembled cassette 302 rearwards into engagement with the main pump unit is occurring, the outlet tube 306 will continue to be pulled into the slots 808 and 816 in the upper and lower ultrasonic housings 800 and 814, respectively. The tapered portion 305 of the tubing adaptor 301 will slide back into the recessed area 818, as shown in Figure 106. Thus, the installation of the assembled cassette 302 into the main pump unit will automatically engage the outlet tube 306 in position between the ultrasonic transducers 866 and 868. The outlet tube 305 is deformed slightly in the slots 808 and 816 since the width of the slots 808 and 816 is less than the outer diameter of the outlet tube 306. This ensures good contact of the outlet tube 306 with the walls 810 and 812 in the upper ultrasonic housing 800, and thus good contact with the ultrasonic transducers 866 and 868.

When the assembled cassette 302 is pushed fully back into place, the front of the assembled cassette 302 is tilted upwards against the bottom of the pump chassis 370, stretching slightly the outlet tube 306. At this point, the first pair of angled segments 372 and 374 on the bottom of the pump chassis 370 get into the area between the front portion 242 of the slide latch 240 and the front top portion of the assembled cassette 302. The slide latch 240 may then be pushed into the cassette body 100 as shown in Figure 106, sliding the inverted L-shaped portion 250 of the slide latch 240 into engagement with the angled segment 372, and sliding the inverted, backwards L-shaped portion 252 of the slide latch 240 into engagement with the angled segment 374. The assembled cassette 302 will thus be held in position on the bottom of the pump chassis 370 until the slide latch 240 is again pulled back, releasing the assembled cassette 302.

Simultaneously, the outlet tube 306 will be opened, but fluid will not flow through the outlet tube 306 since at least one of the valve actuators 620 will be in its fully downward position at any given time, thereby preventing free flow through the assembled cassette 302 whenever the assembled cassette 302 is installed on the main pump unit. It will also be noted that in this initially installed position, the piston cap portion 262 is located at the very top of the pump cylinder 112.

The pumping operation of the system described above will not be described fully. Rather, for a complete description of the pumping operation, EP-A-319277 may be referred to.

Through the above discussion of the entire system, it will be appreciated that the present invention provides an ultrasonic air-in-line detection sensor for use with the outlet tubing from a cassette in which the tubing is automatically loaded into the proper position in the sensor when the cassette is installed on a main pump unit. The system of the present invention thus loads the tubing into the sensor, in the proper position, with no more effort required than the effort to load the cassette itself onto the main pump unit. The tubing is thereby loaded correctly into the sensor in a highly repeatable manner whenever the cassette is installed.

Once the tubing has been properly loaded into the sensor, it is retained in the sensor in a manner making it impossible for the tubing to move with respect to the sensor. The tubing is thus both properly loaded initially into the sensor, and retained in its proper position in the sensor to prevent false signals due to movement of the tubing in the sensor. In addition, the tubing is automatically removed from the sensor when the cassette is removed from the main pump unit without requiring any additional steps to be performed.

Despite the inclusion of these features, the system of the present invention employs a minimum number of parts, all of which are of inexpensive construction, yet which afford the assembled cassette a high degree of accuracy. The system of the present invention is therefore able compete economically with known systems, and it provides an ease of use rivalling the best of these systems. The system accomplishes all these objects in a manner which retains and enhances the advantages of reliability, durability, and safety of operation, without incurring any relative disadvantage.

## Claims

1. A system for automatically loading a segment of tubing (306) extending from a disposable, fluid-pumping cassette (302) into engagement with a sensor as the cassette (302) is installed onto a main pump unit, comprising a main pump unit chassis (370) defining thereunder a location into which the cassette may be installed and a sensor housing (800,814) mounted on the chassis (370) downstream of the location into which the cassette may be installed, characterised in that the sensor housing has a vertically orientated slot (808,816) located at the front for receiving the segment of tubing (306) and a recess (818) located in the bottom of the sensor housing (814) on both sides of the slot (816) in the sensor housing; the system further including a hollow cylindrical tubing adaptor (301) adapted to be mounted on the segment of tubing (306) extending from the bottom of the cassette (302), the top end of the tubing adaptor being tapered (305) in order to be able to engage the recess (818) in the bottom of the sensor housing (814) when the cassette (302) is inserted into the position defined by the main pump unit chassis.

2. A system as claimed in Claim 1, characterised by an ultrasonic transmitter (866) located in the sensor housing (800) on one side of the slot (808); an ultrasonic receiver (868) located in the sensor housing (800) on the other side of the slot (808).

3. A system as claimed in Claim 2, characterised in that the ultrasonic transmitter (866) is adhesively mounted to the interior of the sensor housing (800) on one side of the slot (808), and the ultrasonic receiver (868) is adhesively mounted to the interior of the sensor housing (800) on the other side of the slot (808).

4. A system as claimed in Claim 1 or Claim 2, characterised by an inclined ramp (820,822) located on the side of the sensor housing (814) facing away from the cassette installation location and in the front of the ultrasonic sensor housing on each side of the slot (816), with ramps (820,822) being inclined towards the recess in the ultrasonic sensor housing to urge the tapered end (305) of the tubing adaptor into engagement with the recess (818) in the sensor housing.

5. A system as claimed in any preceding Claim, characterised in that the cassette installation location is beneath the main pump unit chassis (370); the sensor (800,814) housing is mounted on the main pump unit chassis (370) below that location; the slot (808,816) is at the front of the sensor housing and is vertically orientated; the recess (818) is located in the bottom of the sensor housing (814) on both sides of the slot; and the hollow cylindrical tubing adaptor (301) extends from the bottom of the cassette (302), the top end of the tubing adaptor being tapered (305), the tubing adaptor (301) being spaced away from the bottom of the cassette (302) sufficiently far so that as the cassette is inserted into its installation location, the tapered top end (305) of the tubing adaptor engages the bottom of the sensor housing (814), the tapered top end of the tubing adaptor engaging the recess (818) in the bottom of the sensor housing.

6. A system as claimed in any preceding Claim, characterised in that the slot has a funnel-shaped entrance to allow the segment of tubing (306) to be directed into the slot.

7. A system as claimed in any preceding Claim, characterised in that the slot (808,816) is narrower than the outer diameter of the segment of tubing (306).

8. A system as claimed in any preceding Claim, characterised in that the sensor housing comprises: an upper sensor housing (800) which is rectangular in configuration and has a vertically orientated opening (808) at the front forming the upper portion of the slot, the upper housing being open at the bottom, and defining first and second areas in its interior on each side of the opening (808), a third area being defined by that portion of the interior of the upper housing (800) into which the opening (808) does not protrude; and a lower sensor housing (814) located beneath the upper sensor housing (800), the lower sensor housing (814) having an opening (816) at the front forming the lower portion of the slot, the lower sensor housing (814) sealing the open areas of the upper sensor housing (800).

9. A system as claimed in Claim 8, characterised in that the ultrasonic transmitter (866) is located in the first area against the interior wall of first area facing the opening in the upper sensor housing (800), and the ultrasonic receiver (868) is located in the second area against the interior wall of the second area facing the opening in the upper sensor housing (800).

10. A system as claimed in Claim 9, characterised by a first foam segment (878) located between the ultrasonic transmitter (866) and the interior wall of the first area remote from the opening in the upper sensor housing (800); and a second foam segment (878) located between the ultrasonic receiver (868) and the interior wall of the second area remote from the opening in the upper sensor housing (800).

## Patentansprüche

1. Anlage zum automatischen Verbinden eines von einer einmal verwendbaren, eine Flüssigkeit pumpenden Kassette (302) ausgehenden Schlauchabschnitts (306) mit einem Sensor, wenn die Kassette (302) auf einer Hauptpumpeneinheit montiert wird, umfassend ein Chassis (370) der Hauptpumpeneinheit, das unter sich eine Stelle besitzt, an der die Kassette eingesetzt werden kann, und ein Sensorgehäuse (800, 814), das auf dem Chassis (370) unterhalb von der Stelle, an der die Kassette eingesetzt werden kann, angebracht ist, dadurch gekennzeichnet, daß das Sensorgehäuse einen vertikal ausgerichteten, auf der Vorderseite angeordneten Schlitz (808, 816) aufweist, der den Schlauchabschnitt (306) aufnimmt, und eine Ausnehmung (818), die im Boden des Sensorgehäuses (814) auf beiden Seiten des Schlitzes (816) in dem Sensorgehäuse angeordnet ist; wobei die Anlage des weiteren einen hohlen zylindrischen Schlauchadapter (301) umfaßt, der auf dem vom Boden der Kassette (302) ausgehenden Schlauchabschnitt (306) angebracht werden kann, wobei sich das obere Ende des Schlauchadapters (302) verjüngt, um in die Ausnehmung (818) im Boden des Sensorgehäuses (814) eingreifen zu können, wenn die Kassette (302) in die durch das Chassis der Hauptpumpeneinheit definierte Position eingesetzt wird.

2. Anlage nach Anspruch 1, **gekennzeichnet durch** einen Ultraschallsender (866), der in dem Sensorgehäuse (800) auf einer Seite des Schlitzes (808) angeordnet ist; und einen Ultraschallempfänger (868), der in dem Sensorgehäuse (800) auf der anderen Seite des Schlitzes (808) angeordnet ist.

3. Anlage nach Anspruch 2, **dadurch gekennzeichnet, daß** der Ultraschallsender (866) auf einer Seite des Schlitzes (808) auf die Innenseite des Sensorgehäuses (800) geklebt ist, und daß der Ultraschallempfänger (868) auf der anderen Seite des Schlitzes (808) auf die Innenseite des Sensorgehäuses (800) geklebt ist.

4. Anlage nach Anspruch 1 oder Anspruch 2, **gekennzeichnet durch** eine geneigte Rampe (820, 822), die auf der Seite des Sensorgehäuses (814) weg von der Stelle, an der die Kassette eingesetzt ist, und vor dem Gehäuse des Ultraschallsensors auf beiden Seiten des Schlitzes (816) angeordnet ist, wobei die Rampen (820, 822) in Richtung zu der Ausnehmung in dem Gehäuse des Ultraschallsensors geneigt sind, um das verjüngte Ende (305) des Schlauchadapters in Eingriff mit der Ausnehmung (818) in dem Sensorgehäuse zu drücken.

5. Anlage nach einem der vorhergehenden Ansprüche, **dadurch gekennzeichnet, daß** die Stelle, an der die Kassette eingesetzt ist, unterhalb des Chassis (370) der Hauptpumpeneinheit liegt; das Sensorgehäuse (800, 814) auf dem Chassis (370) der Hauptpumpeneinheit unterhalb dieser Stelle angebracht ist; der Schlitz (808, 816) auf der Vorderseite des Sensorgehäuses liegt und vertikal ausgerichtet ist; die Ausnehmung (818) im Boden des Sensorgehäuses (814) auf beiden Seiten des Schlitzes angeordnet ist; und der hohle zylindrische Schlauchadapter (301) vom Boden der Kassette (302) ausgeht, wobei das obere Ende des Schlauchadapters verjüngt ist (305), der Schlauchadapter (301) so weit vom Boden der Kassette (302) angeordnet ist, daß beim Einsetzen der Kassette in ihre Position das verjüngte obere Ende (305) des Schlauchadapters mit dem Boden des Sensorgehäuses (814) in Eingriff kommt, wobei das verjüngte obere Ende des Schlauchadapters in die Ausnehmung (818) im Boden des Sensorgehäuses eingreift.

6. Anlage nach einem der vorhergehenden Ansprüche, **dadurch gekennzeichnet, daß** der Schlitz eine trichterförmige Öffnung besitzt, durch die der Schlauchabschnitt (306) in den Schlitz eingeführt werden kann.

7. Anlage nach einem der vorhergehenden Ansprüche, **dadurch gekennzeichnet, daß** der Schlitz (808, 816) schmaler ist als der Außendurchmesser des Schlauchabschnitts (306).

8. Anlage nach einem der vorhergehenden Ansprüche, **dadurch gekennzeichnet, daß** das Sensorgehäuse folgendes umfaßt: ein oberes Sensorgehäuse (800), das rechteckig ausgebildet ist und auf der Vorderseite eine vertikal ausgerichtete Öffnung (808) besitzt, die den oberen Abschnitt des Schlitzes darstellt, wobei das obere Gehäuse unten offen ist und in seinem Inneren auf beiden Seiten der Öffnung (808) einen ersten und einen zweiten Bereich besitzt, wobei ein dritter Bereich durch denjenigen Abschnitt der Innenseite des oberen Gehäuses (800) gebildet wird, in den die Öffnung (808) nicht ragt; und ein unteres Sensorgehäuse (814), das unterhalb des oberen Sensorgehäuses (800) angeordnet ist, wobei das untere Sensorgehäuse (814) auf der Vorderseite eine Öffnung (816) besitzt, die den unteren Abschnitt des Schlitzes darstellt, wobei das untere Sensorgehäuse (814) die offenen Bereiche des oberen Sensorgehäuses (800) verschließt.

9. Anlage nach Anspruch 8, **dadurch gekennzeichnet, daß** der Ultraschallsender (866) in dem ersten Bereich an der zu der Öffnung in dem oberen Sensorgehäuse (800) weisenden Innenwand des ersten Bereichs angeordnet ist, und daß der Ultraschallempfänger (868) in dem zweiten Bereich an der zu der Öffnung in dem oberen Sensorgehäuse (800) weisenden Innenwand des zweiten Bereichs angeordnet ist.

10. Anlage nach Anspruch 9, **gekennzeichnet durch** ein erstes Schaumstoffstück (878), das zwischen dem Ultraschallsender (866) und der Innenwand des ersten Bereichs weg von der Öffnung in dem oberen Sensorgehäuse (800) angeordnet ist; und ein zweites Schaumstoffstück (880), das zwischen dem Ultraschallempfänger (868) und der Innenwand des zweiten Bereichs weg von der Öffnung in dem oberen Sensorgehäuse (800) angeordnet ist.

## Revendications

1. Système pour charger automatiquement un segment d'un tube (306) s'étendant depuis une cassette jetable de pompage de fluide (302) engagée avec un capteur, tandis que la cassette (302) est installée sur un ensemble principal de pompe, comprenant un châssis d'ensemble principal de pompe (370), définissant sous celui-ci un emplacement dans lequel la cassette peut être installée, et un logement de capteur (800, 814) monté sur le châssis (370), en aval de l'emplacement dans lequel la cassette peut être installée, caractérisé en ce que le logement de capteur comporte une fente orientée verticalement (808, 816) située à l'avant, pour recevoir le segment de tube (306), et une cavité (818) située au fond du logement de capteur (814) des deux côtés de la fente (816) dans le logement de capteur; le système comportant en outre un adaptateur de tube cylindrique creux (301) susceptible d'être monté sur le segment du tube (306) s'étendant depuis le fond de la cassette (302), l'extrémité supérieure de l'adaptateur de tube étant effilée (305) afin de pouvoir s'engager dans la cavité (818) située dans le fond du logement de capteur (814) lorsque la cassette (302) est introduite dans la position définie par le châssis d'ensemble principal de pompe.

2. Système selon la revendication 1, caractérisé par un émetteur à ultrasons (866) situé dans le logement de capteur (800), d'un côté de la fente (808); un récepteur à ultrasons (868) situé dans le logement de capteur (800) de l'autre côté de la fente (808).

3. Système selon la revendication 2, caractérisé en ce que l'émetteur à ultrasons (866) est monté de façon collée à l'intérieur du logement de capteur (800), d'un côté de la fente (808), et le récepteur à ultrasons (868) est monté de façon collée à l'intérieur du logement de capteur (800) de l'autre côté de la fente (808).

4. Système selon la revendication 1 ou la revendication 2, caractérisé par une rampe inclinée (820, 822), situé sur le côté du logement de capteur (814) tourné à l'opposé de l'emplacement d'installation de la cassette et devant le logement de capteur à ultrasons de chaque côté de la fente (816), laquelle rampe (820, 822) étant inclinée vers la cavité située dans le logement de capteur à ultrasons afin de pousser l'extrémité effilée (305) de l'adaptateur de tube en engagement dans la cavité (818) située dans le logement de capteur.

5. Système selon l'une quelconque des revendications précédentes, caractérisé en ce que l'emplacement d'installation des cassettes se trouve en dessous du châssis d'ensemble principal de pompe (370); le logement de capteur (800, 814) est monté sur le châssis d'ensemble principal de pompe (370) en dessous de cet emplacement; la fente (808, 816) se trouve devant le logement de capteur, et elle est orientée verticalement; la cavité (818) est située au fond du logement de capteur (814) des deux côtés de la fente; et l'adaptateur de tube cylindrique creux (301) s'étend depuis le fond de la cassette (302), l'extrémité supérieure de l'adaptateur de tube étant effilée, l'adaptateur de tube (301) étant espacé suffisamment loin du fond de la cassette (302) pour que, lorsque la cassette est introduite dans son emplacement d'installation, l'extrémité supérieure effilée (305) de l'adaptateur de tube s'engage dans le fond du logement de capteur (814), l'extrémité supérieure effilée de l'adaptateur de tube s'engageant dans la cavité (818) située au fond du logement de capteur.

6. Système selon l'une quelconque des revendications précédentes, caractérisé en ce que la fente comporte une entrée en forme d'entonnoir pour permettre au segment du tube (306) d'être dirigé dans la fente.

7. Système selon l'une quelconque des revendications précédentes, caractérisé en ce que la fente (808, 816) est plus étroite que le diamètre extérieur du segment du tube (306).

8. Système selon l'une quelconque des revendications précédentes, caractérisé en ce que le logement de capteur comprend: un logement supérieur de capteur (800) qui est de configuration rectangulaire et comporte une ouverture orientée verticalement (808) à l'avant, formant la partie supérieure de la fente, le logement supérieur étant ouvert au fond, et définissant une première et une seconde surfaces dans son intérieur de chaque côté de l'ouverture, une troisième surface étant définie par la partie de l'intérieur du logement supérieur (800) dans laquelle l'ouverture (808) ne fait pas saillie; et un logement inférieur de capteur (814) situé en dessous du logement supérieur de capteur (800), le logement inférieur de capteur (814) comportant une ouverture (816) à l'avant, formant la partie inférieure de la fente, le logement inférieur de capteur (814) fermant les surfaces ouvertes du logement supérieur de capteur (800).

9. Système selon la revendication 8, caractérisé en ce que l'émetteur d'ultrasons (866) est situé dans la première surface contre la paroi intérieure de la première surface tournée vers l'ouverture située dans le logement supérieur de capteur (800), et le récepteur d'ultrasons (868) est situé dans la seconde surface contre la paroi intérieure de la seconde surface tournée vers l'ouverture située dans le logement supérieur de capteur (800).

10. Système selon la revendication 9, caractérisé par un premier segment de mousse (878) situé entre l'émetteur d'ultrasons (866) et la paroi intérieure de la première surface éloignée de l'ouverture située dans le logement supérieur de capteur (800); et un second segment de mousse (878) situé entre le récepteur d'ultrasons (868) et la paroi intérieure de la seconde surface éloignée de l'ouverture située dans le logement supérieur de capteur (800).
